(19) **Europäisches Patentamt**
**European Patent Office**
**Office européen des brevets**

(11) **EP 1 669 744 B1**

(12) **EUROPÄISCHE PATENTSCHRIFT**

(45) Veröffentlichungstag und Bekanntmachung des
Hinweises auf die Patenterteilung:
**10.09.2008 Patentblatt 2008/37**

(51) Int Cl.:
*G01N 25/70* *(2006.01)*

(21) Anmeldenummer: **04405766.9**

(22) Anmeldetag: **10.12.2004**

(54) **Verfahren zur Messung eines Partialdrucks und Vorrichtung zur Durchführung des Verfahrens**

Method and device for measuring a partial pressure

Méthode et dispositif pour mesurer une pression partielle

(84) Benannte Vertragsstaaten:
**DE FR GB SE**

(43) Veröffentlichungstag der Anmeldung:
**14.06.2006 Patentblatt 2006/24**

(73) Patentinhaber: **Gmeiner, Paul**
**8966 Oberwil-Lieli (CH)**

(72) Erfinder: **Gmeiner, Paul**
**8966 Oberwil-Lieli (CH)**

(56) Entgegenhaltungen:
**CH-A- 469 259** **DE-B- 1 033 443**
**US-A- 2 715 836**

• **WENHAO ZHUANG ET AL: "An automated high pressure PVT apparatus for continuous recording of density and isothermal compressibility of fluids" REVIEW OF SCIENTIFIC INSTRUMENTS AIP, Bd. 67, Nr. 1, Januar 1996 (1996-01), Seiten 244-250, XP002320147 USA ISSN: 0034-6748**

**Beschreibung**

TECHNISCHES GEBIET

Bei der Erfindung wird ausgegangen von einem Verfahren zur Messung des Partialdrucks einer Komponente eines in einem Trocknungsprozess bei Unterdruck anfallenden Gemischs aus einem Inertgas und dem Dampf einer Flüssigkeit resp. aus einem Inertgas, einem Dampf einer höher siedenden Flüssigkeit und einem Dampf einer tiefer siedenden Flüssigkeit. Die Erfindung bezieht sich auch auf eine Vorrichtung zur Durchführung des Verfahrens. Messverfahren der vorgenannten Art werden beim Trocknen, vor allem von elektrischen Isolierungen und solche Isolierungen enthaltenden Hochspannungsapparaten und -maschinen, wie insbesondere Transformatoren, Durchführungen, Kondensatoren oder rotierenden Maschinen, eingesetzt.

Für ein ökonomisches Trocknen sind hierbei Unterdrücke erforderlich, die typischerweise im Bereich von 0,01 bis 10 mbar liegen. Während der Trocknung, speziell gegen Ende der Vakuumphase, ist es wichtig zu wissen, welche Vakuumwerte vorliegen, und wie gross speziell der Wasserdampfpartialdruck wie auch der Luftpartialdruck im Vakuumbehälter ist, damit der Verlauf der Trocknung, insbesondere das Trocknungsende, beurteilt werden kann.

Die Messung und die Feststellung der zeitlichen Abnahme der relevanten Vakuumwerte, respektiv der Partialdrücke, wird bei der herkömmlichen Umluft-Vakuumtrocknung, wie auch bei der in den letzten Jahren hauptsächlich applizierten Solventdampftrocknung durchgeführt. Bei der Solventdampftrocknung wird das Trocknungsobjekt mittels Kondensation eines Solventdampfes aufgeheizt, dies im Gegensatz zur Umluft-Vakuumtrocknung, bei welcher die Erwärmung des Trocknungsobjektes mittels Konvektion durch heisse Luft erfolgt. Demzufolge ist es bei der Solventdampftrocknung ebenfalls vorteilhaft, wenn während der Trocknung nebst dem Totaldruck, dem Wasserdampfpartialdruck und Luftpartialdruck ebenfalls der Solventdampfpartialdruck bestimmt werden kann.

STAND DER TECHNIK

Messverfahren der eingangs genannten Art werden seit langem ausgeführt. Dabei werden Totaldrücke im Bereich von 0.05- 2 mbar, mit Pirani-Messgeräten oder mit gasartunabhängigen Messgeräten mit verschiedenen Messprinzipien, wie Piezo, Dehnstreifen, Kapazitätsänderung usw., festgestellt. Bei einem Pirani-Messgerät ist die Messgenauigkeit des Totaldrucks speziell bei tieferen Vakuumwerten stark von der Gaszusammensetzung beeinflusst. Mit Kompressionsvakuummetern nach Mc.Leod oder nach dem Kammerer-Prinzip kann der Partialdruck von Luft gemessen werden zusammen mit dem dem vorliegenden Kompressionsdruck entsprechenden Partialdruck nicht kondensierbarer Dämpfe. Das heisst bei tieferen Drücken, unterhalb des Verflüssigungsdruckes von Wasser oder Solvent, kann der Luftpartialdruck respektiv der Wasserdampfpartialdruck und oder der Solventdampfpartialdruck nur als Summe und nicht einzeln gemessen werden. Da die Kompressionsvakuummeter handbetrieben sind und zudem Quecksilber als Kompressionsmedium benutzen, ist der Einsatz dieser Geräte aus Gründen des Umwelt und Personenschutzes nicht mehr zeitgemäss.

Der Partialdruck von Wasserdampf kann indirekt auch mit Taupunktmessgeräten ermittelt werden. Diese Messung ist im allgemeinen jedoch verhältnismässig ungenau. Für die Messung eines Wasserdampfpartialdrucks von beispielsweise 0.08 mbar ergibt sich ein Taupunkt von ca. - 44°C, für die eines um 25% reduzierten Wasserdampfpartialdrucks von 0,06 mbar ein Taupunkt von ca. - 45,6°C, was einer Taupunktserniedrigung von lediglich -1,6°C entspricht. Da die Messgenauigkeit kommerziell erhältlicher Taupunktmessgeräte 2 bis 3°C beträgt, kann eine solche Taupunktserniedrigung nicht mehr in befriedigender Weise erfasst werden. Zudem sind kommerziell erhältliche Taupunktmessgeräte im Messbereich für solch tiefe Taupunkte sehr verschmutzungsanfällig.

Darüber hinaus kann der Wasserdampfpartialdruck indirekt durch Adsorption des Wasserdampfs aus dem Gemisch mit Hilfe eines Adsorptionsmittels gemessen werden. Spezialgeräte zur Ausführung dieses Messverfahrens sind in der Firmenbroschüre VZ402 der Fa. Micafil AG, Zürich/Schweiz angegeben. Die Messgenaugkeit bei tiefen Wasserdampfpartialdrücken (< 0.3 mbar) ist jedoch relativ ungenau. Zudem können mit diesem Gerät die Partialdrücke von Luft und Solvent nicht ermittelt werden.

Im Patent CH 469259 A ist ein Verfahren beschrieben, bei welchem der Druck eines Trocknerabgases in einer Messkammer mittels Kompression erhöht und danach die relative Fenchte mit einem Fenchtemessgerät gemessen wird.

Weiterhin ist aus US 2715836 eine Vorrichtung zur Bestimmung von Taupunkt und relatives Fenchte bekannt, bei der die bis zum Erreichen der Kondensation erforderliche Volumenverkleinerung gemessen wird.

DARSTELLUNG DER ERFINDUNG

Der Erfindung, wie sie in den Patentansprüchen angegeben ist, liegt die Aufgabe zu Grunde, ein Messfahren der eingangs genannten Art anzugeben sowie eine Vorrichtung zur Durchführung dieses Verfahren, welche es in einfacher und kostengünstiger Weise ermöglichen, in einem Druckbereich von 0,001 bis 100 mbar mit guter Genauigkeit sowohl den Totaldruck zu ermitteln, als auch im Messbereich zwischen 0,02 und 2 mbar den Partialdruck jeder der Komponenten des Gemischs.

Bei einer ersten Ausführungsform des Verfahrens nach der Erfindung, bei der das Gemisch ein Inertgas und den Dampf lediglich einer Flüssigkeit enthält, wird das Gemisch in einer evakuierbaren Messkammer durch Verkleinern des Volumens der Messkammer komprimiert und hierbei der Verlauf des Totaldrucks und der Temperatur des Gemischs in Abhängigkeit von einem Kompressionsfaktor erfasst, der durch das Verhältnis der Grösse des Messkammervolumens vor der Kompression zur Grösse des Messkammervolumens nach der Kompression bestimmt ist. Zugleich wird der Kompressionsvorgang mindestens solange fortgeführt bis Kondensation des Dampfs einsetzt, und wird aus hierbei ermittelten Werten des Totaldrucks, der Temperatur und des Kompressionsfaktors zumindest beim und/oder nach dem Einsetzen der Kondensation sowie gegebenenfalls dem Totaldruck vor der Kondensation und/oder dem Kondensationsdruck des Dampfs bei der Kondensationstemperatur der Partialdruck der Komponente ermittelt.

Bei einer zweiten Ausführungsform des Verfahrens nach der Erfindung, bei der das Gemisch ein Inertgas, ein Dampf einer höher siedenden Flüssigkeit und einen Dampf einer tiefer siedenden Flüssigkeit enthält, wird das in einer evakuierbaren Messkammer durch Verkleinern des Volumens der Messkammer mindestens solange komprimiert bis Kondensation der höher siedenden Flüssigkeit eintritt und hierbei der Verlauf des Totaldrucks und der Temperatur des Gemischs in Abhängigkeit von einem Kompressionsfaktor erfasst, der durch das Verhältnis der Grösse des Messkammervolumens vor der Kompression zur Grösse des Messkammervolumens nach der Kompression bestimmt ist. Hierbei wird entweder der Kompressionsvorgang mindestens solange fortgeführt bis Kondensation des Dampfs der tiefer siedenden Flüssigkeit einsetzt, und aus hierbei ermittelten Werten des Totaldrucks und des Kompressionsfaktors zumindest beim und/oder nach dem Einsetzen der Kondensation des Dampfs der tiefer siedenden Flüssigkeit sowie gegebenenfalls dem Totaldruck vor der Kondensation und/oder dem Kondensationsdruck des Dampfs der höher und/oder des Dampfs der tiefer siedenden Flüssigkeit bei der Kondensationstemperatur der Partialdruck der Komponente ermittelt. Oder es wird alternativ am komprimierten Gemisch vor der Kondensation des Dampfs der tiefer siedenden Flüssigkeit der Taupunkt des Dampfs der tiefer siedenden Flüssigkeit gemessen und hieraus und aus dem Kompressionsfaktor bei der Taupunktsmessung der Partialdruck des Dampfs ermittelt.

Die erste Phase der Kompression wird so gesteuert, dass noch keine Kondensation des Dampfes der tiefer siedenden Flüssigkeit, beispielsweise Wasser auftritt, sondern nur Kondensation des Dampfes der höher siedenden Flüssigkeit, beispielsweise Solvent. In dieser ersten Phase der Kompression kann alternativ durch Messung des Taupunktes des Dampfes der niedriger siedenden Flüssigkeit indirekt der Partialdruck dieses Dampfes sowie auch derjenige des Inertgases und des Dampfes der höher siedenden Flüssigkeit ermittelt werden. Da Totaldruck und Temperatur des Gas/Dampfgemisches wie auch der Kompressionsfaktor dauernd gemessen werden, kann zudem durch Vergleich des nach der ersten Kompression gemessenen Kompressionsfaktors mit dem effektiven, gemessenen Verhältnis des Druckes vor und nach der ersten Kompression leicht festgestellt werden, ob und wie viel Dampf der höher siedenden Flüssigkeit, beispielsweise des Solvents, kondensiert ist.

In der zweiten Phase der Kompression wird weiter komprimiert bis der Dampf der tiefer siedenden Flüssigkeit, beispielsweise Wasser, weitgehend auskondensiert ist. Mit Hilfe des Verhältnisses zwischen dem nach der zweiten Kompression gemessenen Kompressionsfaktors und dem effektivem Druckverhältnis vor und nach der zweiten Kompression können der Dampfdruck der tiefer siedenden Flüssigkeit, beispielsweise der Wasserdampfpartialdruck, sowie der Partialdruck des Gases, insbesondere von Luft, oder aber auch der Partialdruck des Dampfes der höher siedenden Flüssigkeit, etwa eines Solvents, ermittelt werden.

Alternativ kann nach der zweiten Kompression durch Vergleich des Druckanstieges respektiv der Änderung der Steigung der Druckkurve versus den Kompressionsfaktor, festgestellt werden, bei welchen Werten des Kompressionsfaktoren die Steigung der Druckkurve sich abflacht. Bei der ersten Aenderung der Steigung beginnt die Kondensation des Dampfes der höher siedenden Flüssigkeit. Bei der zweiten Aenderung der Steigung beginnt die Kondensation des Dampfes der tiefer siedenden Flüssigkeit. Basierend auf den gemessenen Kompressionsfaktoren und den der Temperatur entsprechenden physikalischen Dampfdrücken der höher und tiefer siedenden Flüssigkeiten können die Partialdrücke der Dämpfe der höher und tiefer siedenden Flüssigkeiten der beiden Dämpfe sowie des Inertgases ermittelt werden.

Beide Ausführungsformen des Messverfahrens nach der Erfindung zeichnen sich dadurch aus, dass die Vakuummesswerte infolge der Kompression im höherem Bereich liegen und deshalb robuste gasartunabhängige Standard-Vakuummessgeräte eingesetzt werden können, welche bei allfälliger Verschmutzung noch eine gute Messgenauigkeit aufweisen.

Die Ausführung des erfindungsgemässen Verfahrens kann dadurch erleichtert werden, dass das Einsetzen der Kondensation durch Überwachung des Verlaufs des Totaldrucks in Abhängigkeit vom Kompressionsfaktor bestimmt wird. Diese Überwachung kann sehr einfach in einer die Messdaten speichernden und verarbeitenden Mess- und Rechnereinheit ausgeführt werden.

Eine die Durchführung des erfindungsgemässen Verfahrens erleichternde Vorrichtung weist eine Messkammer auf, deren Volumen durch die Position eines lageveränderlichen Kolbens bestimmt ist. Mit Vorteil begrenzt dieser Kolben zwei Arbeitsräume begrenzt, von denen der eine die Messkammer und der andere eine Druckausgleichskammer ist. Eine Reduktion der Messgenauigkeit durch kleine Undichtheiten der Messkammer kann nun sehr einfach dadurch vermieden werden, dass der bei der Kompression in der Messkammer aufgebaute Druck durch einen in einer Ausgleichskammer wirkenden Gegendruck weitgehend kompensiert wird.

Ist bei einer Ausführungsform der erfindungsgemässen Vorrichtung der Messkammer eine erste Vorkompressionskammer vorschaltbar und wird in dieser Kammer das Gemisch vor dem Einbringen in die Messkammer kondensationsfrei komprimiert, so kann diese Messvorrichtung durch Einsatz einer kostengünstigen Messkammer wesentlich vereinfacht werden. Zugleich kann durch die Vorkompression die Genauigkeit des erfindungsgemässen Verfahrens erheblich erhöht werden. Grosse Kompressionsfaktoren und dementsprechend eine hohe Messgenauigkeit können erreicht werden, wenn der Messkammer mindestens eine zweite Vorkompressionskammer vorschaltbar ist, welche parallel zur ersten Vorkompressionskammer geschaltet ist.

Grundsätzlich kann beim erfindungsgemässen Verfahren adiabatisch komprimiert werden, da durch kontinuierliche Druck- und Temperaturüberwachung sowie die Erfassung des Kompressionsfaktors der thermodynamische Zustand des Gemisches jederzeit erfasst ist und aus den erfassten Daten die Partialdrücke der Gemischkomponenten über die Zustandsgleichung des Gemischs die Partialdrücke ermittelt werden können. Aus Gründen einer einfacheren Datenverarbeitung und einer Reduktion der Messparameter empfiehlt es sich jedoch, das Gemisch vor und/oder während der Kompression zu kühlen und es somit während des Messverfahrens auf konstanter Temperatur zu halten. Eine zur Durchführung dieses isotherm ablaufenden Verfahrens geeignete Vorrichtung nach der Erfindung weist daher mit Vorteil ein temperaturüberwachten Kühlelement auf, welches mit der Messkammer in Wirkverbindung steht.

Das Messverfahren nach der Erfindung wird in vorteilhafter Weise bei einem beim Trocknen von elektrischen Isolationen und/oder von Apparaten mit elektrischen Isolationen in einer Vakuumtrockenkammer anfallenden Gas-Dampf-Gemisch angewendet, welches mit mindestens einer Vakuumpumpe aus dieser Kammer entfernt wird. Aus den ermittelten Werten des Partialdrucks des Dampfs resp. der Partialdrücke des Dampfs der tiefer und des Dampfs der höher siedenden Flüssigkeit sowie der Saugleistung der Vakuumpumpe kann dann leicht die pro Zeiteinheit anfallende Menge an abgepumpter Flüssigkeit resp. an abgepumpter höher und abgepumpter tiefer siedender Flüssigkeit ermittelt wird.

Wird das erfindungsgemässe Messverfahren beim Trocknen von elektrischen Isolationen und/oder von Apparaten mit elektrischen Isolationen in einer Vakuumtrockenkammer eingesetzt, so wird mit Vorteil in der Vakuumtrockenkammer zusätzlich mindestens ein Probestück verwendet, welches aus dem Material der Isolationen besteht und einen mit der Vakuumkammer kommunizierenden Hohlraum aufweist, der zur Messung des Partialdrucks mindestens einer der Komponenten eines im Hohlraum befindlichen Gas-Dampf-Gemischs mit der Messkammer verbunden wird.

Hierbei wird das zu messende Gas-Dampf-Gemisch durch Zurückziehen des Kompressionskolbens in die Messkammer eingebracht. Die Messung des Partialdrucks und eine zusätzliche Messung der Temperatur, jeweils im Inneren des Probestücks, stellen in vorteilhafter Weise sicher, dass die Abnahme des Flüssigkeitsgehalts der elektrischen Isolationen leicht beurteilt und dementsprechend der Trocknungsverlauf kontinuierlich überwacht werden kann.

KURZE BESCHREIBUNG DER FIGUREN

Anhand von Zeichnungen werden nachfolgend Ausführungsbeispiele der Erfindung näher erläutert. Hierbei zeigen die

Figuren 1 bis 5 zur Trocknung von Hochspannungsapparaten vorgesehene Vorrichtungen, in die jeweils eine von fünf Ausführungsformen einer Messvorrichtung zur Durchführung des Verfahrens nach der Erfindung eingebaut ist,

Fig.6    ein Diagramm, in dem der Verlauf des Totaldrucks und der Partialdrücke eines Gemischs aus einem Inertgas L und dem Dampf lediglich einer Flüssigkeit W in Funktion eines das Gemisch mit Druck beaufschlagenden Kompressionsfaktors dargestellt ist, und

Fig.7    ein Diagramm, in dem der Verlauf des Totaldrucks und der Partialdrücke eines Gemischs aus einem Inertgas L, dem Dampf einer niedriger siedenden Flüssigkeit W und dem Dampf einer höher siedenden Flüssigkeit S in Funktion des Kompressionsfaktors dargestellt ist.

WEGE ZUR AUSFÜHRUNG DER ERFINDUNG

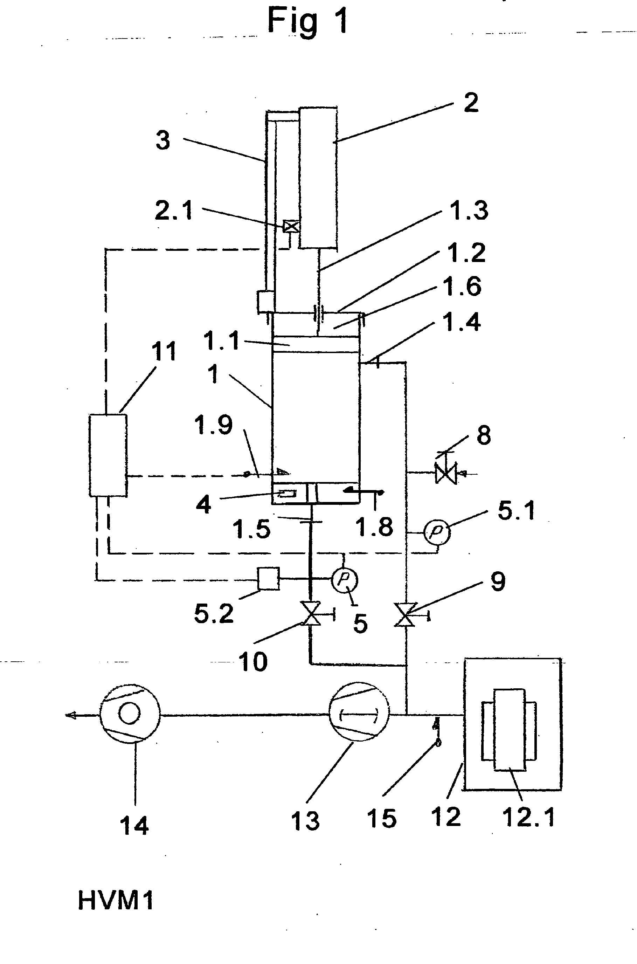

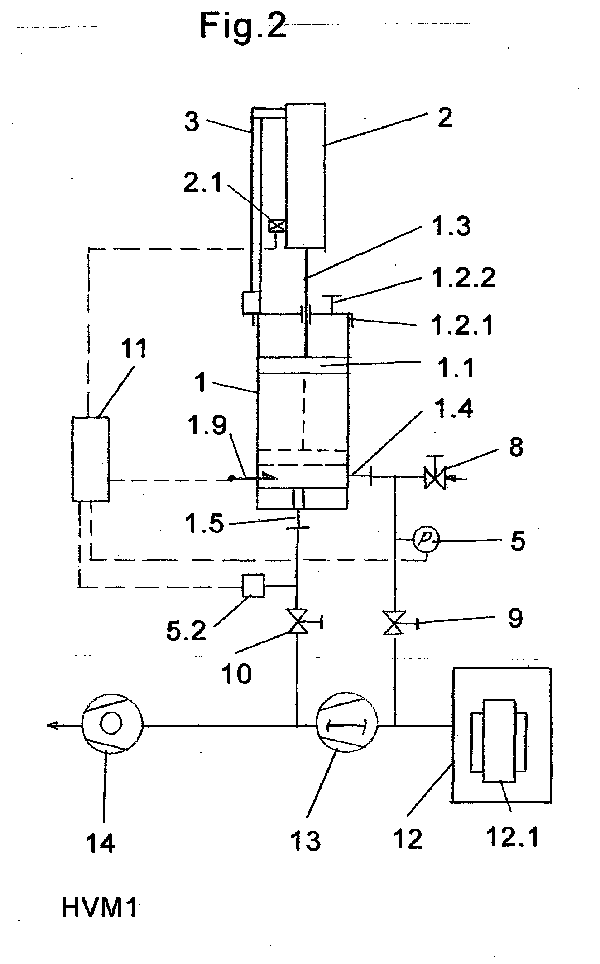

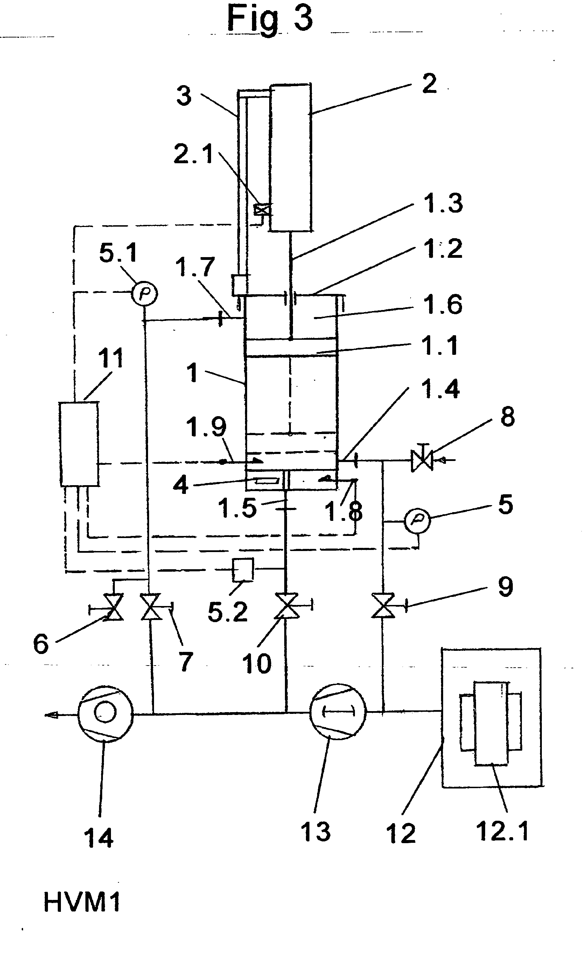

Die in den Figuren 1 bis 5 dargestellten fünf Ausführungsformen des erfindungsgemässen Messsystems dienen der Messung von Totaldrücken von 100- 0.001 mbar sowie der Partialdrücke von Gas/Dampfgemischen im UnterdrucklVakuumbereich von 2- 0.02 mbar. Mit den Absperrventilen (9;10) wird das Messsystem mit der Vakuum-Trockenkammer (12) und den Vakuumpumpen (13;14) verbunden. In der Vakuum Trockenkammer (12) ist das Trocknungsobjekt (12.1), welches während dem Trocknungsprozess mit den Vakuumpumpen (13; 14) unter Vakuum gehalten wird ,damit die in der Isolation vorhandene Feuchtigkeit und das entsprechend der Trocknungstechnologie (Solventdampftrocknung) allfällig vorhandene Solvent abgedampft werden kann.

Das Messsystem nach Fig.1 besteht aus einer vakuum und druckdichten Messkammer (1) mit einem verschiebbarem Kompressionskolben (1.1) welcher eine nicht gezeichnete vakuum und druckfeste Dichtung enthält, welche den Kommpressionsraum der Messkammer (1) vakuum und druckdicht von der Ausgleichskammer (1.6) abtrennt. Die Messkammer (1) ist mit einem Kühlelement (4) zum Beispiel einem Peltierelement , sowie Temperatursensoren (1.8) für die Messkammer und den Gasraum (1.9) ausgerüstet. Die mit der Messkammer (1) verbundene Antriebseinheit (2) bewegt die Kolbenstange (1.3) und den angekoppelten Kompressionskolben (1.1).Mit Hilfe der elektronischen Distanzmessung (3) und dem beweglichen, elektronischen Sensor (2.1) wird die Verschiebungsdistanz das heisst der Kompressionsfaktor (K. F) bestimmt. Mit dem Totaldruck Vakuummessgerät (5) wird der Totaldruck in der Messkammer (1) vor und nach der Kompression und der Kühlung des Gas/Dampfgemisches ermittelt. Basierend auf den gemessen Kompressionsfaktoren (KF1;KF1.1;KF1,2;KF2) und den mit dem Vakuummessgerät (5) gemessenen Drücken vor und nach der Kompression sowie der Gas/Dampftemperatur (1.9), wird in der Mess/ Rechner-Einheit (11) basierend auf den physikalischen Grundlagen (Dampfdruckkurven) errechnet, ob eine Kondensation von Dampf der höher siedenden Flüssigkeit beispielsweise Solventdampf oder der tiefer siedenden Flüssigkeit beispielsweise Wasserdampf aufgetreten ist und wie gross der Partialdruck des kondensierten Solventdampfes, wie auch des Wasserdampfes und der Luft war. Basierend auf den gemessenen und errechneten Wasserdampfpartialdrücken (POW) des Gas/Dampfgemisches in der Messkammer (1) vor der Kompression, welcher identisch ist mit den Wasserdampfpartialdrücken in der Vakuum-Trockenkammer (12) kann unter Berücksichtigung der Temperatur des Gas/Dampfgemisches in der Messkammer (1.9) und in der Saugleitung (15) die zeitlich abgepumpte Wassermenge errechnet werden, falls die Saugleistung der Vakuumpumpen (13;14) bekannt ist.

Ein alternativ eingebautes Taupunktmessgerät (5.2) kann den Taupunkt des Dampfes nach der Kompression des Gas/Dampfgemisches messen, sofern keine Kondensation von Dampf auftrat und mit Hilfe des ermittelten Kompressionsfaktors kann der Taupunkt respektiv der Wasserdampfpartialdruck des in die Messkammer(1) eingesaugten Gas/Dampfgemisches bestimmt werden.

Mit den Absperrventilen (8;9) kann über die Eintrittsöffnung (1.4;1.7) der Druck in der Ausgleichskammer (1.6) während der Kompression auf gleichem Niveau wie in der Messkammer (1) gehalten und mit Totaldruckmessgerät (5.1) kontrolliert werden. Ueber die Eintrittsöffnung (1.4) und Absperrventil (8) wird Spülluft der Messkammer (1) zugeführt und über die Ablassöffnung (1.5) und Ventil (10) den Vakuumpumpe (13;14) zugeleitet und abgepumpt.

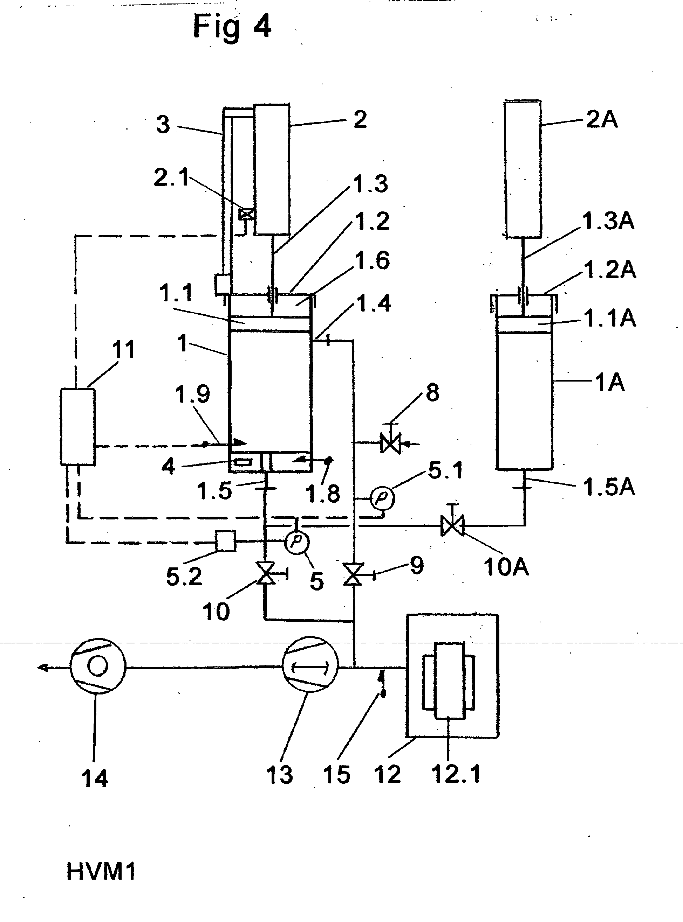

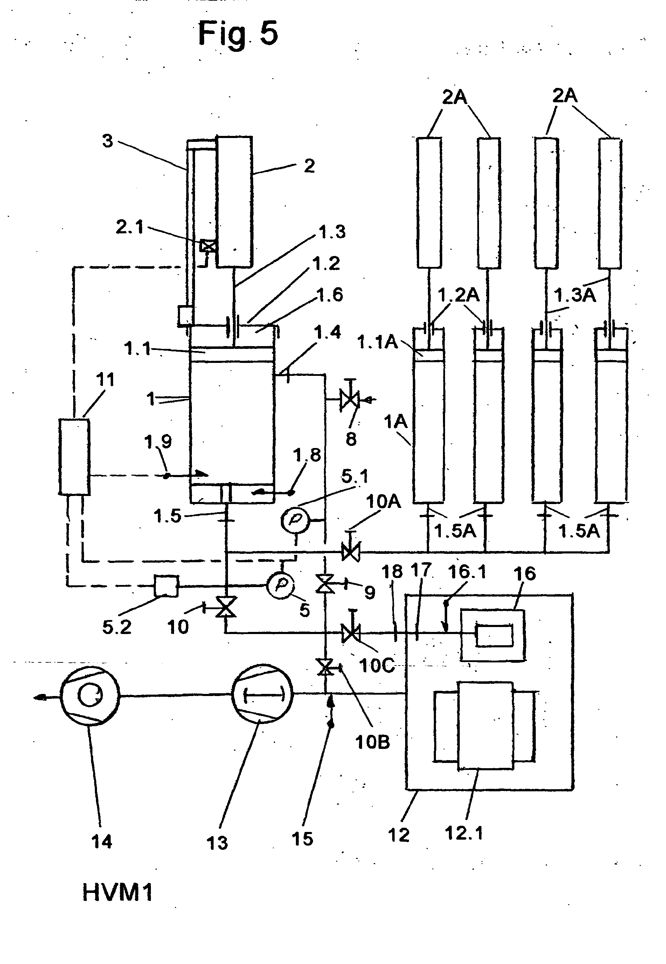

Mit einer Messapparatur gemäss Fig.4 & 5 kann das in die Messkammer (1) einströmende Gas/Dampfgemisches in mindestens einer Vorkompressionskammer (1A) vorkomprimiert werden. Dabei wird über die geöffneten Absperrventile (10;10A) (Fig.4) resp. (10; 10A, 10C) oder (10, 10A,10B) (Fig.5) das Gas/Dampfgemisch durch Zurückziehen der Kompressionskolben (1.1;1.1A) in die Messkammer (1) und Vorkompressionskammer (1A) eingezogen. Anschliessend wird bei zurückgezogenem Kompressionskolben(1.1) mit dem Vorkompressionskolben (1.1A) über die Ablassöffnung (1.5A) und dem geöffneten Absperrventil (10A) das Gas/Dampfgemisch in die Messkammer (1) gepresst.Während der anschliesend erfolgten Kompression in der Messkammer (1) ist das Absperrventil (10A) geschlossen und der Kompressionskolben (1.1) wird nach unten bewegt.Dadurch werden in der Messkammer (1) wesentlich höhere Kompressionsfaktoren erreicht, was eine genauere Erfassung der Partialdrücke sicherstellt. Zudem können einfache handelsübliche Kompressions respektiv Messkammern eingesetzt werden. Im weiteren wird durch die Vorkompressionskammern (1A) das sich während der Vorkompression erwärmte Gas/Dampfgemisch gekühlt. Dadurch kann auf eine aktive Kühlung des Gases in der Messkammer (1) verzichtet werden.

Der Kompressionsfaktor darf nur so gross sein, dass keine Kondensation von Dämpfen während der Vorkompression erfolgt, da ansonsten dass Messresultat leicht verfälscht würde.

Alternativ kann mit einer Messapparatur gemäss Fig.1 - 5 ebenfalls, die Partialdrücke eines Gas/Dampfgemisches im Innern, eines in der Vakuum Trockenkammer (12) liegenden Probestückes (16) ermittelt werden. Basierend auf der ebenfalls gemessenen Temperatur und der aus der einschlägigen Literatur bekannten Korrelationsfaktoren, zwischen Wasserdampfpartialdruck, Temperatur versus Feuchtegehalt, kann der Feuchtegehalt in der Isolation des Probestückes ermittelt werden. Dabei wird über die Verbindungsleitungen (17;18) und Absperrventil (10C) das im Innern eines Probestückes (16) vorhandene Gas/Dampfgemisch in der vorgängig beschriebenen Art und Weise in die Messapparatur eingebracht und die Partialdrücke ermittelt.

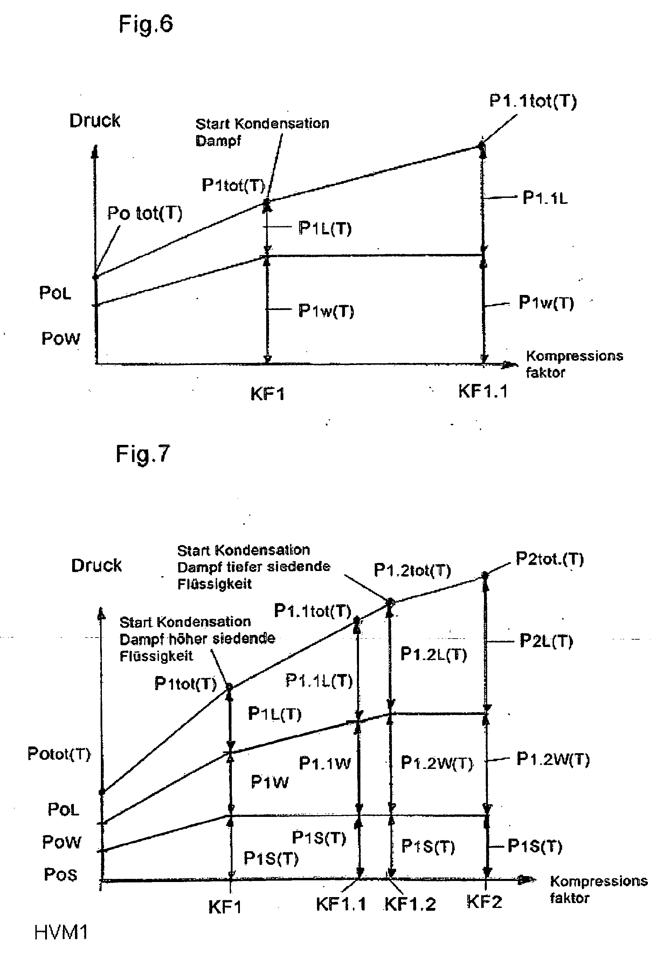

Die Ermittlung von Totaldruck und Partialdrücken von Komponenten eines Gas/Dampfgemisches welches während beispielsweise einer Vakuum-Trocknung eines elektrischen Gerätes (12.1) aus der Vakuum- Trockenkammer (12) oder aus dem Innern eines Probestückes (16) abgepumpt wird und beispielsweise aus Wasserdampf sowie Luft besteht, ist anhand des nachfolgenden Messzyklus MZ01, des Druckverlaufdiagramms (Fig.6) und der Legende (L1) in 2 Varianten dargestellt. Bei Variante 1 wird das Gas/Dampfgemisches soweit komprimiert bis Kondensation von Wasserdampf auftritt. Bei Variante 2 wird das Gas/Dampfgemisches nur soweit komprimiert, dass keine Kondensation von Wasserdampf auftritt. Dabei wird während der Kompression der Taupunkt und die Gas/Dampftemperatur gemessen.

Variante 1: Kompression mit Kondensation von Wasserdampf (wird anhand eines Berechnungsbeispiels und Messzyklus MZ01 dargestellt).

Messzyklus MZ01

Spülen der Messkammer (1) und Vorkompressionskammer(1.1) mit Frischluft über die geöffneten Absperrventile (8) und (10;10A). Der Kompressionskolben (1.1;1.1A) ist in rückgezogener Position, sodass das gesamte Messkammervolumen (1) und Vorkompressionskammervolumen (1A) mit Frischluft gespült wird.

- Ausstossen der Spülluft mit Kompressionskolben (1.1;1.1A) über geöffnetes Absperrventil (10;10A;10B) bei geschlossenen Absperrventilen (8) und (9). Die Spülluft wird mit Vakuumpumpen (13;14) abgepumpt.

- Einströmen des zu messenden, abgepumpten Gasgemisches von der Vakuum-Trockenkammer (12) oder Probestück (16) durch Zurückziehen des Kompressionskolben (1.1;1.1A) über das geöffnete Absperrventil (10;10A;10B_ oder 10C) in die Messkammer (1) und Vorkompressionskammer (1A) bei geschlossenen Absperrventilen(8;9).

- Druckausgleich in Messkammer (1) und Vorkompressionskammer (1A) mit der Vakuum-Trockenkammer (12) oder Probestück (16) bei gleichzeitiger Abkühlung des Gas-Dampfgemisches auf eine Temperatur, welche oberhalb der Kondensationstemperatur von Wasserdampf ($T_{OW}(P)$) liegt und Messung der Temperatur des Gas/Dampfgemisches (To) mit Temperatur Sensor Gasraum (1.9) und Temperatursensor (15) oder (16.1) sowie alternativer Messung des Totaldruckes (Po tot(T)) in der Messkammer (1)

- Vorkompression des Gas-Dampfgemisches bei geöffnetem Abspenventil (10a) mit Kompressionskolben (1.1A) falls vorhanden.

- Kompression des Gas/Dampfgemisches in Messkammer (1) bei geschlossenem Absperrventil (10, 10A) mit Kompressionskolben (1.1) auf einen Druck, welcher oberhalb des Kondensationsdruckes von Wasser liegt, oder alternativ bis zum max.zulässigen Kompressionsfaktor (KFm), bei gleichzeitiger Kühlung des Gasgemisches mit Kühlelement (4) auf die vorgegebene Temperatur ($T_0$) und kontinuierlicher Messung der Kompressionsfaktoren(KF1;KF1.1). Ueber Ventil (7 oder 9 oder 8) wird ebenfalls die Ausgleichskammer (1.6) auf dem gleichen Druck wie die Messkammer (1) gehalten.
Falls trotz hohem Kompressionfaktor und hohem Kondensationsdruck, keine Kondensation von Wasserdampf auftritt, das heisst ein sehr kleiner Wasserdampfpartialdruck (Pow) vorliegt, so kann dies durch Vergleich der gemessenen Totaldrücke $P_{1.1\,tot(T)}$ und $P_{otot(T)}$ in Relation zum Kompressionsfaktor (KF1.1) wie folgt festgestellt werden:
Falls KF1.1 = ($P_{1.1tot(T)}$) / ($P_{o.tot(T)}$) so liegt keine Kondensation von Wasserdampf vor. Der in diesem Fall vorliegende, kleine Wasserdampfpartialdruck, kann von $P_{1W(T)}$ = 0 bis zum max. Wert von $P_{1W(T)}$. varieren. Der max. mögliche Wasserdampfpartialdruck wäre demzufolge : $P_{OW}$ = $P_{1W(T)}$ / KF1.1
Falls KF1.1 < ($P_{1.1tot(T)}$ / ($P_{o.tot(T)}$) liegt ein Leck in der Messkammer (1) vor, da diese Konstellation physikalisch bei isothermer Kompression nicht möglich ist.
Falls KF1.1 > ($P_{1.1tot(T)}$ / ($P_{o.tot(T)}$) so kondensiert Wasserdampf.
Die Berechnung der Partialdrücke($P_{OW}$,$P_{OL}$) und Totaldruck ($P_{Otot(T)}$) kann nach veschiedenen Alternativen erfol-gen.

<u>Alternative 1</u>

Kompression gemäss Diagramm (Fig.6) bis Kompressionsfaktor (KF1;KF1.1) und Gastemperatur $T_1$ =$T_{1.1}$= To - Berechnung des Luftpartialdruckes ($P_{OL}$), des Wasserdampfpartialdruckes (Pow) und des Totaldruckes ($P_{Otot\,(T)}$) on der Messkammer (1) respektiv in der Vakuum - Trockenkammer (12) oder Probestück (16), durch die Mess/Rechner-Einheit (11) wie folgt :
Berechnung von Totaldruck ($P_{o.t(T)}$) in Messkammer vor Kompression

$$P_{o.t(T)} = (P_{1.1\,tot(T)} + P_{1.1\,w(T)} * (KF1.1 / KF1 - 1)) / KF1.1$$

Berechnungsbeispiel :  -Kompressionsfaktor KF1.1 = 400(A)
Vorgabe = (A)  -Kompressionsfaktor KF1 = 200 (G)
Gemessen = (G)  (Start Kondensation H2O)
  -Totaldruck in Messkammer $P_{1.1\,tot(T)}$= 56.6 mbar (G)

(fortgesetzt)

- Gas/Dampftemperatur $T_{1.1}$ = 20 °C
- Gas/Dampftemperatur To = 20 °C
- Wasserdampfdruck bei 20°C ; $P_{1.1\ w(T)}$ =23.37 mbar

$$P_{o.tot} = (56.6 + 23.37 * (400/200 - 1)) / 400 = 0.2\ mbar$$

Berechnung von Luftpartialdruck ($P_{o.L}$) in Messkammer vor Kompression

$$P_{o.L} = ( P_{1.1\ tot(T)} - P_{1.1\ w(T)} ) / KF1.1$$

$$P_{o.L} = (56.6 - 23.37 ) / 400 = 0.083\ mbar$$

Berechnung von Wasserdampfpartialdruck ($P_{o.w}$) in Messkammer vor Kompression

$$P_{o.w} = (P_{1.1\ tot(T)} + P_{1.1\ w(T)} * (KF1.1/ KF1 - 1 )) / KF1.1 - P_{o.L}$$

$$P_{o.w} = (56.6 + 23.37 * (400/200 - 1 ) ) / 400 - 0.083\ mbar = 0.117\ mbar$$

Alternative 1.1

Kompression gemäss Diagramm (Fig.6) bis Kompressionsfaktor (KF1;KF1.1) und Gastemperatur $T_1 = T_{1.1} >$ To

Berechnung von Totaldruck ($P_{o.tot}$) in Messkammer vor Kompression:

$$P_{o.t(T)} = ((To/T1.1)^{(\kappa/\kappa-1)}) * (P_{1.1\ tot(T)} + P_{1.1\ w(T)} * ( KF1.1/ KF1 - 1)) / KF1.1$$

Berechnungsbeispiel : -Kompressionsfaktor KF1.1 = 400 (A)
Vorgabe (A) -Kompressionsfaktor KF1 = 362.6 (G)
Gemessen (G) (Start Kondensation H2O bei 30 °C)
-Totaldruck in Messkammer $P_{1.1\ tot(T)}$= 85.3 mbar (G)
- Gas/Dampftemperatur $T_{1.1}$ = 30 °C+ 273° =303°K(A)
- Wasserdampfdruck bei 30°C ; $P_{1.1\ w(T)}$ = 42.43 mbar
-mittl.Adiabatenexponent $\kappa$ = 1.35 (A)

$$P_{o.t(T)} = ((293/303)^{(1.35/1.35-1)}) * (85.3 + 42.43 * (400/362.6 - 1))/400 \doteq 0.2\ mbar$$

Berechnung von Luftpartialdruck ($P_{o.L}$) in Messkammer vor Kompression:

$$P_{o.L} = ( P_{1.1\ tot(T1.1)} * (To/T1.1)^{(\kappa/\kappa-1)} - P_{1.1\ w(T1.1)} ) / KF1.1$$

$$P_{o.L} = (85.3\ mbar * (293/303)^{(1.35/1.35-1)} - 42.43\ mbar ) / 400 \doteq 0.083\ mbar$$

Berechnung von Wasserdampfpartialdruck (Po.w) in Messkammer vor Kompression:

$$P_{o.w} = ((T_o/T1.1)^{(\kappa/\kappa-1)}) * (P_{1.1\,tot(T)} + P_{1.1\,w(T)} \cdot (KF1.1/KF1 - 1)) / KF1.1 - P_{o.L}$$

$$P_{o.w} = ((293/303)^{(1.35/1.35-1)}) * (85.3 + 42.43 * (400/362.6 - 1))/400 - 0.081 = 0.117 \text{ mbar}$$

<u>Alternative 2</u>

Kompression gemäss Diagramm (Fig.6) und Messung von Kompressionsfaktor (KF1; KF1.1) und Totaldruck $P_{otot(T)}$; Gastemperatur $T_1 = T_{1.1} = T_o$
- Berechnung des Wasserdampfpartialdruckes ($P_{OW}$) & Luftpartialdruck (POL) in der Messkammer (1) respektiv in der Vakuum Trockenkammer (12) oder Probestück (16), durch die Mess/Rechner-Einheit (11) wie folgt:
Berechnung von Wasserdampfpartialdruck (Po.w) in Messkammer vor Kompression

$$P_{o.w} = (P_{o\,tot} * KF1.1 - P_{1.1\,tot(T1.1)}) / (KF1.1 - KF1)$$

Berechnungsbeispiel :    -Totaldruck in Messkammer $P_{otot(T)}$ = 0.2 mbar (G)
Vorgabe (A)    -Totaldruck in Messkammer $P_{1.1\,tot(T)}$ = 56.6 mbar (G)
Gemessen (G)    - Kompressionsfaktor KF1.1 = 400 (A)
   -Kompressionsfaktor KF1 = 200 (G)
   (Start Kondensation H2O )
   - Wasserdampfdruck bei 20°C ; $P_{1.1\,w(T)}$ = 23.37 mbar

$$P_{o.w} = (0.2 * 400 - 56.6) / (400 - 200) = 0.117 \text{ mbar}$$

Berechnung von Luftpartialdruck ($P_{o.L}$) in Messkammer vor Kompression

$$P_{o.L} = (P_{1.1\,tot(T)} - P_{1w\,(T)}) / KF1.1$$

$$P_{o.L} = (56.6 - 23.37) / 400 = 0.083 \text{ mbar}$$

<u>Alternative 2.1</u>

Kompression gemäss Diagramm (Fig.6) und Messung von Kompressionsfaktor (KF1;KF1.1 ) und Totaldruck $P_{otot(T)}$; Gastemperatur $T_1 = T_{1.1} > T_o$
Berechnung von Wasserdampfpartialdruck (Po.w) in Messkammer vor Kompression

$$P_{o.w} = ((P_{o\,tot} * KF1.1 - (P_{1.1\,tot(T1.1)} - P_{1w\,(T1.1)}) / (KF1.1-KF1)) * (T_{1.1}/T_o)^{(\kappa/\kappa-1)}) - P_{1.1\,tot(T1.1)}) / (KF1.1 - KF1)$$

Berechnungsbeispiel :    -Kompressionsfaktor KF1.1 = 400 (A)
Vorgabe (A)    -Kompressionsfaktor KF1 = 362.6 (G)
Gemessen (G)    (Start Kondensation H2O bei 30 °C)
   - Totaldruck in Messkammer $P_{otot(T)}$ = 0.2 mbar (G)
   -Totaldruck in Messkammer $P_{1.1\,tot(T)}$ = 85.3 mbar (G)

(fortgesetzt)

- Gas/Dampftemperatur $T_{1.1} = 30\,°C + 273° = 303\,°K$ (A)
- Wasserdampfdruck bei 30°C ; $P_{1.1\ w(T)} = 42.43$ mbar
- mittl. Adiabatenexponent $\kappa = 1.35$ (A)

$$P_{o.w} = \left((0.2 * 400 - (85.3 - 42.43)/(400-362.6) * (303/293)^{(1.35/1.35-1)}) - 85.3\right) / (400 - 362.6) \doteq 0.117 \text{ mbar}$$

Berechnung von Luftpartialdruck ($P_{o.L}$) in Messkammer vor Kompression

$$P_{o.L} = (P_{1.1\ tot(T1.1)} * (T_o/T_{1.1})^{(\kappa/\kappa-1)} - P_{1w\ (T1.1)}) / KF1.1$$

$$P_{o.L} = (85.3 * (293/303)^{(1.35/1.35-1)} - 42.43) / 400 \doteq 0.083 \text{ mbar}$$

### Alternative 3

Kompression gemäss Diagramm (Fig.6) und Messung von Kompressionsfaktor (KF1.1) und Totaldruck $P_{otot(T)}$; Gastemperatur $T_{1.1} = T_o$ ; Wasserdampfkondensationsdruck $P_{1w(T1)}$ gemäss T1.1
- Berechnung des Wasserdampfpartialdruckes ($P_{OW}$) & Luftpartialdruck (POL) in der Messkammer (1) respektiv in der Vakuum -Trockenkammer (12) oder Probestück (16), durch die Mess/Rechner-Einheit (11) wie folgt :
Berechnung von Wasserdampfpartialdruck ($P_{o.w}$) in Messkammer vor Kompression

$$P_{o.w} = (P_{o\ tot(T)} * KF1.1 - P_{1.1\ tot(T)} + P_{1w(T)}) / KF1.1$$

| Berechnungsbeispiel : | -Kompressionsfaktor KF1.1 = 400 (A) |
| Vorgabe (A) | - Totaldruck in Messkammer $P_{otot(T)}$ = 0.2 mbar (G) |
| Gemessen (G) | -Totaldruck in Messkammer $P_{1.1\ tot(T)}$ = 56.6 mbar (G) |
| | - Wasserdampfdruck bei 20°C ; $P_{1.1}\ w(T)$ = 23,.37 mbar |

$$P_{o.w} = (0.2*400 - 56.6 + 23.37) / 400 = 0.117 \text{ mbar}$$

Berechnung von Luftpartialdruck ($P_{o.L}$) in Messkammer vor Kompression

$$P_{o.L} = (P_{1.1\ tot(T)} - P_{1w(T)}) / KF1.1$$

$$P_{o.L} = (56.6 - 23,.37) / 400 = 0.083 \text{ mbar}$$

### Alternative 3.1

Kompression gemäss Diagramm (Fig.6) und Messung von Kompressionsfaktor (KF1.1) und Totaldruck $P_{otot(T)}$; Gastemperatur $T_{1.1} > T_o$ ; Wasserdampfkondensationsdruck $P_{1w(T1)}$ gemäss T1.1
Berechnung von Wasserdampfpartialdruck ($P_{o.w}$) in Messkammer vor Kompression

$$P_{o.w} = (P_{o\ tot(T)} * KF1.1 - P_{1.1\ tot(T)} * (T_o/T_{1.1})^{(\kappa/\kappa-1)} + P_{1w(T1.1)}) / (KF1.1)$$

| Berechnungsbeispiel | -Kompressionsfaktor KF1.1 = 400 (A) |
|---|---|
| Vorgabe (A) | - Totaldruck in Messkammer $P_{otot(T)}$ = 0.2 mbar (G) |
| Gemessen (G) | -Totaldruck in Messkammer $P_{1.1\ tot(T)}$ = 85.3 mbar (G) |
| | - Gas/ Dampftemperatur $T_{1.1}$ = 30 °C+ 273° = 303 °K (A) |
| | -Wasserdampfdruck bei 30°C ; $P_{1.1\ w(T)}$ = 42.43 mbar |
| | -mittl.Adiabatenexponent K = 1.35 (A) |

$$P_{o.w} = ((0.2*400 - 85.3* (293/303)^{(1.35/1.35-1)} + 42.43) / (400) \tilde{=} 0.117\ mbar$$

Berechnung von Luftpartialdruck ($P_{o.L}$) in Messkammer vor Kompression

$$P_{o.L} = (P_{1.1\ tot(T)} * (T_o/T_{1.1})^{(\kappa/\kappa-1)} - P_{1.1\ w(T)}) / KF1.1$$

$$P_{o.L} = 85.3 *(293/303)^{(1.35/1.35-1)} - 42.43 ) / 400 \tilde{=} 0.083\ mbar$$

### Alternative 4

Kompression gemäss Diagramm (Fig.6) und Messung von Kompressionsfaktor (KF1) bei Start Kondensation Wasserdampf;Totaldruck in Messkammer $P_{1\ tot(T)}$; Gastemperatur $T_1 = T_O$ ; Wasserdampfkondensationsdruck $P_{1w(T1)}$ gemäss T1
- Berechnung des Wasserdampfpartialdruckes ($P_{OW}$) & Luftpartialdruck ($P_{OL}$) in der Messkammer (1) respektiv in der Vakuum -Trockenkammer (12) oder Probestück (16), durch die Mess/Rechner-Einheit (11) wie folgt :
Berechnung von Wasserdampfpartialdruck (Po.w) in Messkammer vor Kompression

$$P_{o.w} = (P_{1w(T1)}) / KF1$$

| Berechnungsbeispiel: | -Kompressionsfaktor KF1 = 200 (G) |
|---|---|
| Vorgabe (A) | -Totaldruck in Messkammer $P_{1\ tot(T1)}$ = 40 mbar (G) |
| Gemessen (G) | - Wasserdampfdruck bei 20°C ; $P_{1\ w(T1)}$ = 23,.37 mbar |

$$P_{o.w} = (23.37) / 200 = 0.117\ mbar$$

Berechnung von Luftpartialdruck ($P_{o.L}$) in Messkammer vor Kompression

$$P_{o.L} = (P_{1\ tot(T1)} - P_{1w(T1)}) / KF1$$

$$P_{o.L} = (40 - 23.37) / 200 = 0.083\ mbar$$

Alternative 4.1

Kompression gemäss Diagramm (Fig.6) und Messung von Kompressionsfaktor (KF1) bei Start Kondensation Wasserdampf;Totaldruck in Messkammer $P_{1\,tot(T)}$; Gastemperatur $T_1 > T_0$ ; Wasserdampfkondensationsdruck $P_{1w(T1)}$ gemäss T1

Berechnung von Wasserdampfpartialdruck ($P_{o.w}$) in Messkammer vor Kompression:

$$P_{o.w} = (\ P_{1W(T1)}\ )\ /\ KF1_{(T1)}$$

| Berechnungsbeispiel: | -Kompressionsfaktor $KF1_{(T1)}$ = 362.6 (G) |
|---|---|
| Vorgabe (A) | -Totaldruck in Messkammer $P_{1tot(1)}$ = 76.6 mbar (G) |
| Gemessen (G) | - Gas/ Dampftemperatur $T_{1.1}$ = 30 °C+ 273° = 303 °K (A) |
| | - Wasserdampfdruck bei 30°C ; $P_{1.1\,w(T1)}$ = 42.43 mbar |
| | -mittl.Adiabatenexponent $\kappa$ = 1.35 (A) |

$$P_{o.w} = (\ 42.43\ )\ /\ 362.6\ ) \cong 0.117\ mbar$$

Berechnung von Luftpartialdruck ($P_{o.L}$) in Messkammer vor Kompression

$$P_{o.L} = ((\ P_{1\,tot(T1)} - P_{1W(T1)}\ )\ {}^*(T_0/T_{1.1})^{(\kappa/\kappa-1)}\ )\ /\ KF1_{(T1)}$$

$$P_{o.L} = ((76.6 - 42.43\ )\ {}^*(293/303)^{(1.35/1.35-1)}\ )\ /\ 362.6\ ) \cong 0.083\ mbar$$

Variante 2 Messung des Taupunktes (wird anhand eines Berechnungsbeispiels und Messzyklus MZ02 dargestellt):

Messzyklus MZ02

Der Messzyklus MZ02 ist bis zum Start der Kompression des Gas/Dampfgemisches identisch wie unter Variante 1, Messzyklus MZ01 danach erfolgt :

Kompression des Gas/Dampfgemisches in Messkammer (1) mit Kompressionskolben (1.1) bei geschlosenen Absperrventilen (10;10A) auf einen Druck welcher unterhalb des Kondensationsdruckes von Wasserdampf ($P_{1W(T)}$) liegt. Basierend auf der kontinuirlichen Messung von : Totaldruck ($P_{1tot(T)}$); Kompressionsfaktor (KF) ;Temperatur (T) und dem Taupunkt des komprimierten Wasserdampfes (5.2) kann der Wasserdampfpartialdruck des komprimierten Wasserdampfes ($P_{1w}$) ,sowie der Wasserdampfpartialdruck ($P_{ow}$) vor der Kompression in der Messkammer (1) respektiv in der Vakuum Trockenkammer (12) oder im Probestück (16) errechnet werden.

Alternative 1 (Gas/Dampftemperatur T1 = To)

**[0054]** - Berechnung des Wasserdampfpartialdruckes (Pow) und Luftpartialdruck (POL) in der Messkammer (1) respektiv in der Vakuum -Trockenkammer (12) oder Probestück (16), durch die Mess/Rechner-Einheit (11) wie folgt : Berechnung von Wasserdampfpartialdruck (Po.w) in Messkammer vor Kompression:

$$P_{o.w} = (\ P_{1W(TP)}\ )\ /\ KF_{(TPM)}$$

Berechnungsbeispiel: -Kompressionsfaktor $KF_{(T.P.M)}$ = 80 (A)
Vorgabe (A) -Totaldruck in Messkammer $P_{1tot(T)}$ = 16 mbar (G)
Gemessen (G) - Taupunkt: 6 °C (G)
- Wasserdampfdruck bei Taupunkt; $P_{1\,w(TP)}$ = 9.35 mbar

$$P_{o.w} = ( 9.35 ) / 80 = 0.117 \text{ mbar}$$

Berechnung von Luftpartialdruck ($P_{o.L}$) in Messkammer vor Kompression

$$P_{o.L} = ( P_{1\,tot(T1)} - P_{1W(T1)} ) / KF_{(T.P.M)}$$

$$P_{o.L} = ( 16 - 9.35 ) / 80 = 0.083 \text{ mbar}$$

Alternative 1.1 (Gas/Dampftemperatur $T_{(TPM)}$ > $T_O$)

Berechnung des Wasserdampfpartialdruckes ($P_{OW}$) & Luftpartialdruck ($P_{OL}$) in der Messkammer (1) respektiv in der Vakuum -Trockenkammer (12) oder Probestück (16), durch die Mess/Rechner-Einheit (11) wie folgt: Berechnung von Wasserdampfpartialdruck ($P_{o.w}$) in Messkammer vor Kompression:

$$P_{o.w} = ( P_{W(TPM)} ) *(T_o/T_{(TPM)})^{(\kappa/\kappa-1)} / KF_{(TPM)}$$

Berechnungsbeispiel: -Kompressionsfaktor $KF_{(T.P.M)}$ = 80 (A)
Vorgabe (A) -Totaldruck in Messkammer $P_{1tot(T)}$ = 18.2 mbar (G)
Gemessen (G) - Taupunkt: 7.9 °C (G)
- Wasserdampfdruck bei Taupunkt ; Pw (TPM) = 10.65 mbar
- Gas/ Dampftemperatur TTPM = 30 °C+ 273° = 303 °K (A)
-mittl.Adiabatenexponent K = 1.35 (A)

$$P_{o.w} = ( 10.65) *(293/303)^{(1.35/1.35-1)} / ) = 0.117 \text{ mbar}$$

Berechnung von Luftpartialdruck ($P_{o.L}$) in Messkammer vor Kompression

$$P_{o.L} = ( ( P_{tot(TPM)} - P_{W(TPM)} ) *(T_o/T_{(TPM)})^{(\kappa/\kappa-1)} ) / KF_{(T.P.M)}$$

$$P_{o.L} = ( 18.2 - 10.65 ) *(293/303)^{(1.35/1.35-1)} / 80 ) = 0.083 \text{ mbar}$$

Die Ermittlung von Totaldruck und Partialdrücken von Komponenten eines Gas/Dampfgemisches welches während beispielsweise bei einer Solventdampftrocknung (Vapour Phase Trocknung) eines elektrischen Gerätes (12.1) aus der Vakuum -Trockenkammer (12) oder aus dem Innern eines Probestückes (16) abgepumpt wird und Dämpfen aus einer tiefer siedenden Flüssigkeit beispielsweise Solvent und einer höher siedenden Flüssigkeit beispielsweise Wasser sowie Luft besteht, wird in 2 Varianten dargestellt . Bei Variante 1 wird das Gas/Dampfgemisches soweit komprimiert bis Kondensation von Solventdampf und Wasserdampf auftritt. Bei Variante 2 wird das Gas/Dampfgemisches nur soweit komprimiert bis Kondensation von Solventdampf, jedoch keine Kondensation von Wasserdampf auftritt. Dabei wird während der Kompression der Taupunkt des Waserdampfes und die Gas/Dampftemperatur gemessen.

<u>Variante 1</u> (Kompression mit Kondensation von Solventdampf und Wasserdampf)

wird anhand eines Berechnungsbeispiels und Messzyklus MZ1, des Druckverlaufdiagramms Fig.7 und der Legende (L1) dargestellt.

Messzyklus MZ1

- Spülen der Messkammer (1) und Vorkompressionskammer (1.A) mit Frischluft über die geöffneten Absperrventile (8) und (10;10A). Der Kompressionskolben (1.1;1.1A) ist in rückgezogener Position, sodass das gesamte Messkammervolumen (1) und Vorkompressionsvolumen (1A) mit Frischluft gespült wird.

- Austossen der Spülluft aus Messkammer (1) und Vorkompressionskammer (1A) über geöffnetes Absperrventil (10; 10A;10B) bei geschlossenen Absperrventilen (8) und (9). Die Spülluft wird mit Vakuumpumpen (13;14) abgepumpt.

- Einströmen des zu messenden, abgepumpten Gasgemisches von der Vakuum-Trockenkammer (12) oder Probestück (16) durch Zurückziehen des Kompressionskolben (1.1;1.1A) über das geöffnete Absperrventil (10;10A;10B oder 10C) in die Messkammer (1) und Vorkompressionskammer (1A) bei geschlossenen Absperrventilen (8;9).

- Druckausgleich in Messkammer (1) mit der Vakuum-Trockenkammer (12), oder Probestück (16) mit gleichzeitiger Abkühlung des Gas-Dampfgemisches auf eine Temperatur, welche oberhalb der Kondensationstemperatur von Solventdampf ($T_{OS(P)}$) liegt und Messung der Temperatur des Gas/Dampfgemisches (To) mit Temperatur Sensor Gasraum (1.9) und Temperatursensor (15) oder (16.1) sowie alternativer Messung des Totaldruckes (Po tot(T));(5) in der Messkammer (1)

- Vorkompression des Gas-Dampfgemisches bei geöffnetem Absperrventil (10a) mit Kompressionskolben (1.1A) falls vorhanden.

- erste Phase der Kompression des Gas/Dampfgemisches mit Kompressionskolben (1.1) auf einen Druck, welcher unterhalb des Kondensationsdruckes von Wasserdampf, jedoch oberhalb des Kondensationsdruckes von Solventdampf liegt, oder alternativ bis zum maximal zulässigen Kompressionsfaktor KFm, bei gleichzeitiger Kühlung des Gasgemisches mit Kühlelement (4) auf beispielsweise 20°C, sowie kontinuierliche Messung des Kompressionsfaktors.(KF1;KF1.1) Ueber Ventil (7oder 8 oder 9) wird ebenfalls die Ausgleichskammer(1.6) auf dem gleichen Druck wie die Messkammer (1) gehalten.

Falls trotz hohem Kompressionfaktor und hohem Kondensationsdruck, keine Kondensation von Solventdampf auftritt, das heisst ein sehr kleiner Solventdampfpartialdruck (Pos) vorliegt, so kann dies durch Vergleich der gemessenen Totaldrücke $P_{1.1}$ tot(T) und $P_{o\ tot(T)}$ in Relation zum Kompressionsfaktor (KF1.1) wie folgt festgestellt werden:

falls KF1.1 = ($P_{1.1tot(T)}$) / ($P_{o.tot\ (T)}$) so liegt keine Kondensation von Solventdampf vor. Der in diesem Fall vorliegende, kleine Solventdampfpartialdruck, kann von P1S(T) = 0 bis zum max.Wert von $P_{1S(T)}$. variieren. Der max. mögliche Solventdampfpartialdruck wäre demzufolge :

$$Pos = P_{1\ s(T)} / KF1.1$$

Falls KF1.1 < ($P_{1.1tot(T)}$) / ($P_{o\ tot\ (T)}$) liegt ein Leck in der Messkammer (1) vor, da diese Konstellation physikalisch bei isothermer Kompression nicht möglich ist.

Falls KF1.1 > ($P_{1.1tot(T)}$) / ($P_{o\ tot\ (T)}$) so kondensiert Solventdampf.

- zweite Phase der Kompression bis zum max. zulässigen Druck (P.mex), oder alternativ bis zum max. Kompressionsfaktor (KF max) unter gleichzeitiger Kühlung des Gasgemisches durch das Gehäuse der Messkammer oder alternativ mit Kühlelement (4) und Messung der Gastemperatur mit Temperatur Sensor Gasraum (1.9), des Druckes ($P_2$ tot(T)) in Messkammer (1) mit Totaldruck Vakuummessgerät (5) und des Kompressionsfaktors (KF1.2 ; KF2) mit der elektronischen Distanzmessung (3).In der 2.Phase der Kompression wird Solventdampf weiter kondensiert, sowie Wasserdampf falls, der erforderliche Kondensationsdruck für Wasserdampf vorliegt. Tritt trotz hohem Kompressionfaktor und hohem Kondensationsdruck, keine Kondensation von Wasserdampf auftritt, so liegt ein sehr kleiner Wasserdampfpartialdruck ($P_{OW}$) vor. Dies kann durch Vergleich der gemessenen Totaldrücke $P_{2\,tot(T)}$ und $P_{o\,tot(T)}$ in Relation zum Kompressionsfaktor (KF2) wie folgt festgestellt werden:

Falls KF2 = $(P_{2tot(T)} - P_{1S(T)}) / (P_{o\,tot(T)})$ so liegt keine Kondensation von Wasserdampf vor. Der in diesem Fall vorliegende, kleine Wasserdampfpartiatdruck, kann von $P_{1.2W(T)} = 0$, bis zum max.Wert von $P_{1.2W(T)}$. variieren. Der max. mögliche Wasserdampfpartialdruck wäre demzufolge : Pow = P1.2W(T) / KF2.

Falls KF2 < $(P_{2tot(T)} - P_{1S(T)}) / (P_{o\,tot(T)})$ liegt ein Leck in der Messkammer (1) vor, da diese Konstellation physikalisch bei isothermer Kompression nicht möglich ist.

Falls KF2 > $(P_{2tot(T)} - P_{1S(T)} / (P_{o\,tot(T)})$ so kondensiert Wasserdampf.

Alternative 1

Kompression des Gas/dampfgemisches bis Kompressionsfaktor (KF2) ,bei Gastemperatur $T_1 = T_{1.1} = T_{1.2} = T_2 = T_o$
- Berechnung des Luftpartialdruckes ($P_{OL}$), des Wasserdampfpartialdruckes ($P_{OW}$) des Solventdampfpartialdruckes ($P_{OS}$) und des Totaldruckes ($P_{otot\,(T)}$) in der Messkammer (1) respektiv in der Vakuum -Trockenkammer (12) oder im Probestück (16), gemäss Diagramm (Fig7) in diesem Fall durch die Mess/Rechner-Einheit (11) wie folgt:
Berechnung des Luftpartialdrucks ($P_{OL}$) in Messkammer vor Kompression

$$P_{OL} = ( P_{2\,tot(T)} - P_{1S(T)} - P_{1.2W(T)} ) / KF2$$

| | |
|---|---|
| Berechnungsbeispiel : | - Kompressionsfaktor KF 2 = 360 (A) |
| Vorgabe (A) | -Kompressionsfaktor KF1.1 = 60 (A) |
| Gemessen (G) | -Totaldruck in Messkammer $P_{2\,tot(T2)}$ = 40 mbar (G) |
| | -Totaldruck in Messkammer $P_{1.1\,tot(T1.1)}$ = 23 mbar (G) |
| | - Wasserdampfdruck bei 20°C ; $P_{1\,w(T1)}$ = 23.37 mbar |
| | - Solventdampfdruck bei 20°C ; $P_{1\,s(T1)}$ = 0.655 mbar |

$$P_{OL} = ( 40\,mbar - 0.655\,mbar - 23.37\,mbar ) / 360 = 0.044\,mbar.$$

Berechnung Wasserpartialdruckes ($P_{OW(T)}$) in Messkammer vor Kompression

$$P_{OW} = ( P_{1tot\,(T)} - P_{1.1\,S(T)} - (P_{2\,tot(T)} - P_{1\,S(T)} - P_{1.2\,W(T)}) *KF1.1/ KF2 )/ KF1.1$$

$$P_{OW} = ( 23 - 0.655 - ( 40 - 0.655 - 23.37)*60/ 360) / 60 = 0.328\,mbar$$

Alternative 1.1 (Gas/Dampftemperatur $T1 = T_{1.1} = T_{1.2} = T_2 > T_o$)

Berechnung Luftpartialdruck ($P_{OL}$) & Wasserdampfpartialdruck ($P_{OW}$) in der Messkammer (1) respektiv in der Vakuum -Trockenkammer (12) oder im Probestück (16), durch die Mess/Rechner-Einheit (11) wie folgt :
Berechnung von Luftpartialdruck (Po.L) in Messkammer vor Kompression

$$P_{o.L} = (( P_{2\,tot(T)} - P_{1S(T)} - P_{1.2W(T)} ) *(T_o/T_{1.1})^{(\kappa/\kappa-1)} ) / KF2$$

| | |
|---|---|
| Berechnungsbeispiel : | -Kompressionsfaktor KF 2 = 360 (A) |
| Vorgabe (A) | -Kompressionsfaktor KF1.1 = 60 (A) |

(fortgesetzt)

Gemessen (G)
- Totaldruck in Messkammer $P_{2\,tot(T2)}$ = 61.7 mbar (G)
- Totaldruck in Messkammer $P_{1.1\,tot(T1.1)}$ = 26.7 mbar(G)
- Wasserdampfdruck bei 30°C ; $P_{2w(T1)}$ = 42.43 mbar
- Solventdampfdruck bei 30°C ; $P_{1\,s(T1)}$ = 1.23 mbar
- Gas/Dampftemperatur $T_{1.1}$ = 30 °C + 273° = 303°K (A)
- mittl.Adiabatenexponent K = 1.35 (A)

$$P_{o.L} = \left(\left(61.7 - 1.23 - 42.43\right)\ *(293/303)^{(1.35/(1.35-1))}\right)/\,360\,\right) \doteq 0.044 \text{ mbar}$$

Berechnung von Wasserdampfpartialdruck ($P_{o.w}$) in Messkammer vor Kompression:

$$P_{OW} = \left(\left(P_{1.1tot\,(T)} - P_{1.1\,S(T)}\right)\ *(T_o/T_{1.1})^{(K/K-1)} - P_{1.1\,S(T)} - \left(P_{2\,tot(T)}\ *(T_o/T_{1.1})^{(K/(K-1))} - P_{1S(T)} - P_{2w\,(T)}\right)\ *KF1.1/\,KF2\,\right)/\,KF1.1$$

$$P_{OW} = \left(\,(26.7 - 1.23)\ *\ (293/303)^{(1.35/1.35-1)} - 1.23\ -\ (61.7\ *(293/303)^{(1.35/1.35-1)} - 1.23 - 42.43)\ *60/\,360)\right)/\,60 \doteq 0.328 \text{ mbar}$$

Berechnung der stündlichen Wasserabpumpung ($G_{w\,(h)}$)durch die Vakuumpumpen (13;14) wie folgt:

$$G_{w\,(h)} = S\ *\ P_{OW}\ \cdot F_{k}\cdot (T_o/T_a)$$
( Annahme : S = 3000 m3/h ; $F_{k}$ = 0.75 gr/m3,mbar bei 20°C und 1mbar; $T_a$ =373 °K : $T_o$ = 293 °K)
$$G_{w\,(h)} = 3000\ m3/h * 0.328\ mbar \cdot 0.75\ gr/m3,mbar \cdot (293/373) = 579.7\ gr\ H20\ /h.$$

## Alternative 2

Kompression des Gas/Dampfgemisches bis Kompressionsfaktor (KF1.1) und Messung von $P_{Otot.(T)}$ und $P_{1.1\,tot\,(T)}$, bei Gastemperatur $T_1 = T_{1.1} = T_o$

Berechnung Solventdampfpartialdruckes ($P_{OS}$) in der Messkammer (1) respektiv in der Vakuum -Trockenkammer (12) oder im Probestück (16), gemäss Diagramm (Fig.7) durch die Mess/Rechner-Einheit (11) wie folgt::

$$P_{OS} = \left(\,P_{1S(T)} + P_{0\,tot\,(T)}\cdot KF1.1 - P_{1.1\,tot\,(T)}\,\right)/KF1.1$$

Berechnungsbeispiel :
Vorgabe (A)
Gemessen (G)
- Kompressionsfaktor KF1.1 = 60 (A)
- Totaldruck in Messkammer $P_{1.1\,tot(T1.1)}$ = 23 mbar (G)
- Totaldruck in Messkammer $P_{o\,tot(T)}$ = 0.5 mbar (G)
- Solventdampfdruck bei 20°C ; $P_{1\,s(T1)}$ = 0.655 mbar

$$P_{OS} = (0.655\ + 0.5\ *\ 60 - 23\,)/\,60 = 0.128 \text{ mbar}$$

<u>Alternative 2.1</u>

Kompression des Gas/Dampfgemisches bis Kompressionsfaktor (KF1.1) und Messung von $P_{Otot.(T)}$ und $P_{1.1\ tot\ (T)}$, bei Gastemperatur $T_1 = T_{1.1} > T_o$

Berechnung des Solventdampfpartialdruckes (Pos) in der Messkammer (1) respektiv in der Vakuum -Trockenkammer (12) oder im Probestück (16), gemäss Diagramm (Fig.7) durch die Mess/Rechner-Einheit (11) wie folgt:

$$Pos = (\ (P_{0\ tot\ (T)} \cdot KF1.1)\ ^*(T_{1.1}/T_o)^{(\kappa/(\kappa-1))}\ -P_{1.1\ tot\ (T)})\ /KF1.1$$

Berechnungsbeispiel :- Kompressionsfaktor KF1.1 = 60 (A)
Vorgabe (A) -Totaldruck in Messkammer $P_{1.1\ tot(T1.1)}$ = 26.7 mbar (G)
Gemessen (G) -Totaldruck in Messkammer $P_{o\ tot(T)}$= 0.5 mbar (G)
 - Solventdampfdruck bei 30°C ; $P_{1\ s(T1)}$ = 1.23 mbar
 - Gas/ Dampftemperatur $T_{1.1}$ = 30°C+ 273° = 303 °K (A)
 -mittl.Adiabatenexponent $\kappa$ = 1.35 (A)

$$Pos = ((0.5 \cdot 60)^*(303/293)^{(1.35/(1.35-1))}\ -26.7)/60 \stackrel{=}{=} 0.128\ mbar$$

<u>Alternative 3</u>

Kompression des Gas/Dampfgemisches bis Kompressionsfaktor (KF1;KF1.1) und Messung von $P_{Otot.(T)}$ und $P_{1.1}$ tot (T),bei Gastemperatur $T_1 = T_{1.1} = T_o$

Berechnung des Solventdampfpartialdruckes (Pos) in der Messkammer (1) respektiv in der Vakuum -Trockenkammer (12) oder im Probestück (16), gemäss Diagramm (Fig.7) ohne Berücksichtigung temperaturabhängiger Solventdampfdruck durch die Mess/Rechner-Einheit (11) wie folgt::

$$Pos = (\ P_{0\ tot\ (T)} \cdot KF1.1 - P_{1.1\ tot\ (T)})\ /\ (KF1.1 - KF1)$$

Berechnungsbeispiel : - Kompressionsfaktor KF1.1 = 60 (A)
Vorgabe (A) - Kompressionsfaktor KF1 = 5.1 (G)
Gemessen (G) -Totaldruck in Messkammer $P_{1.1\ tot(T1.1)}$ = 23 mbar (G)
 -Totaldruck in Messkammer $P_{o\ tot(T)}$ = 0.5 mbar (G)

$$Pos = (\ 0.5\ ^*\ 60 - 23\ )\ /\ (60 - 5.1) = 0.128\ mbar$$

<u>Alternative 3.1</u>

Kompression des Gas/Dampfgemisches bis Kompressionsfaktor (KF1;KF1.1) und Messung von $P_{Otot.(T)}$ und $P_{1.1\ tot\ (T)}$, bei Gastemperatur $T_1 = T_{1.1} > T_o$

Berechnung Solventdampfpartialdruckes ($P_{OS}$) in der Messkammer (1) respektiv in der Vakuum -Trockenkammer (12) oder im Probestück (16), gemäss Diagramm (Fig.7) ohne Berücksichtigung temperaturabhängiger Solventdampfdruck durch die Mess/Rechner-Einheit (11) wie folgt:

$$Pos = ( P_{0\,tot\,(T)} \cdot KF1.1*(T_{1.1}/T_0)^{(\kappa/(\kappa-1))} - P_{1.1s\,(T)} \cdot (KF1.1-KF1)/KF1 - P_{1.1\,tot\,(T)} ) / (KF1.1 - KF1)$$

| Berechnungsbeispiel : - | Kompressionsfaktor KF1.1 = 60 (A) |
|---|---|
| Vorgabe (A) - | Kompressionsfaktor KF1 = 9.6 (G) |
| Gemessen (G) | -Totaldruck in Messkammer $P_{1.1\,tot(T1.1)}$ = 26.7 mbar (G) |
| | -Totaldruck in Messkammer $P_{o\,tot(T)}$ = 0.5 mbar (G) |
| | - Solventdampfdruck bei 30°C ; $P_{1\,s(T1)}$ = 1.23 mbar |
| | - Gas/ Dampftemperatur $T_{1.1}$ = 30 °C + 273° = 303 °K (A) |
| | -mittl.Adiabatenexponent $\kappa$ = 1.35 (A) |

$$Pos = (0.5 \cdot 60*(303/293)^{(1.35/(1.35-1))} - 1.23 \cdot (60-9.6)/60 - 26.7)/(60-9.6) ) \doteq 0.128 \text{ mbar}$$

Alternative 4

## Kompression des Gas/Dampfgemisches bis Kondensation Solventdampf (KF1) und Kondensation Wasserdampf (KF1.2) bei Gastemperatur $T_1 = T_{1.2} = T_0$

Indirekte Bestimmung des Solventdampfpartialdruckes(Pos), des Wasserdampfpartialdruckes($P_{OW}$), durch Messung des Kompressionsfaktors KF1 (Start Kondensation des Solventdampfes; ist identisch mit der ersten Aenderung der Steigung der Druckkurve), des KF 1.2 (Start Kondensation des Wasserdampfes, ist identisch mit der zweiten Aenderung der Steigung der Druckkurve).

Berechnung des Solventdampfpartialdruckes (Pos)und des Wasserdampfpartialdruckes (Pow) in der Messkammer (1) respektiv in der Vakuum-Trockenkammer (12) oder im Probestück (16) durch die Mess/ Rechner-Einheit (11) wie folgt,

$$Pos = P_{1s(T)} / KF1$$

| Berechnungsbeispiel : - | Kompressionsfaktor KF1 = 5.1 (G) |
|---|---|
| Vorgabe (A) | Solventdampfdruck bei 20°C ; $P_{1\,s(T1)}$ = 0.655 mbar |
| Gemessen (G) | |

$$Pos = 0.655 / 5.1 \doteq 0.128 \text{ mbar}$$

Berechnung des Wasserdampfpartialdruckes(Pow) durch die Mess/ Rechner-Einheit (11) wie folgt:

$$Pow = P_{1.2w(T)} / KF1.2$$

| Berechnungsbeispiel : - | Kompressionsfaktor KF1.2 = 71.3 (G) |
|---|---|

(fortgesetzt)

Vorgabe (A)        -Wasserdampfdruck bei 20°C ; $P_{1\,W(T1)}$ = 23.37 mbar
Gemessen (G)

$$Pow = 23.37 \,/\, 71.3 = 0.328 \text{ mbar}$$

Alternative 4.1

Kompression des Gas/Dampfgemisches bis Kondensation Solventdampf (KF1) und Kondensation Wasserdampf (KF1.2) bei Gastemperatur $T_{1=}T_{1.2} > T_o$

Indirekte Bestimmung des Solventdampfpartialdruckes(Pos), des Wasserdampfpartialdruckes ($_{POW}$), durch Messung des Kompressionsfaktors KF1 (Start Kondensation des Solventdampfes; ist identisch mit der ersten Aenderung der Steigung der Druckkurve), des KF 1.2 (Start Kondensation des Wasserdampfes, ist identisch mit der zweiten Aenderung der Steigung der Druckkurve).

Berechnung des Solventdampfpartialdruckes (Pos)und des Wasserdampfpartialdruckes ($P_{OW}$) in der Messkammer (1) respektiv in der Vakuum -Trockenkammer (12) oder im Probestück (16) durch die Mess/ Rechner-Einheit (11) wie folgt,

$$Pos = P_{1S(T)} \,/\, KF1$$

Berechnungsbeispiel : -    Kompressionsfaktor KF1= 9.6 (G)
Vorgabe (A) -              Solventdampfdruck bei 30°C ; $P_{1\,s(T1)}$ = 1.23 mbar
Gemessen (G)

$$Pos = 1.23 \,/\, 9.6\,) \overset{=}{=} 0.128 \text{ mbar}$$

Berechnung des Wasserdampfpartialdruckes(Pow) durch die Mess/ Rechner-Einheit (11) wie folgt:

$$Pow = P_{1.2W(T)} \,/\, KF1.2$$

Berechnungsbeispiel : -    Kompressionsfaktor KF1.2= 129.4 (G)
Annahme (A) -              Wasserdampfdruck bei 30°C ; $P_{1\,W(T1)}$ = 42.43 mbar
Gemessen (G)

$$Pow = 42.43 \,/\, 129.4\,) \overset{=}{=} 0.328 \text{ mbar}$$

Variante 2

Kompression bis Kondensation von Solventdampf, jedoch keine Kondensation von Wasserdampf und Messung des Wasserdampfes mit Taupunktmessgerät; ist anhand eines Berechnungsbeispiels und Messzyklus MZ2, des Druckverlaufdiagramms Fig.7 und der Legende (L1) dargestellt.

Messzyklus MZ2 :

-   Spülen der Messkammer (1)

-   Ausstossen der Spülluft aus Messkammer

- Einströmen des zu messenden, abgepumpten Gasgemisches

- Druckausgleich in Messkammer (1)

- Vorkompression des Gas-Dampfgemisches wird wie unter Variante 1 beschrieben durchgeführt.

- Kompression des Gas/Dampfgemisches mit Kompressionskolben (1.1) auf einen Druck, welcher unterhalb des Kondensationsdruckes von Wasserdampf, jedoch oberhalb des Kondensationsdruckes von Solventdampf liegt, oder alternativ bis zum maximal zulässigen Kompressionsfaktor KFm, sowie kontinuirliche Messung des Kompressionsfaktors.(KF1;KF1.1) und des Taupunktes des Wasserdampfes mit Taupuktmessgerät (5.2).Ueber Ventil (7oder 8 oder 9) wird ebenfalls die Ausgleichskammer (1.6) auf dem gleichen Druck wie die Messkammer (1) gehalten

Falls trotz relativ hohem Kompressionfaktor und relativ hohem Kondensationsdruck, keine Kondensation von Solventdampf auftritt, das heisst ein sehr kleiner Solventdampfpartialdruck ($P_{OS}$) vorliegt, so kann dies durch Vergleich der gemessenen Totaldrücke $P_{1.1\,tot(T)}$ und $P_{o\,tot(T)}$ in Relation zum Kompressionsfaktor (KF1.1) wie folgt festgestellt werden:

falls $KF1.1 = (P_{1.1tot(T)}) / (P_{o\,tot\,(T)})$ so liegt keine Kondensation von Solventdampf vor. Der in diesem Fall vorliegende, kleine Solventdampfpartialdruck, kann von $P_{1\,S(T)} = 0$ bis zum max.Wert von $P_{1S(T)}$ variieren. Der max. mögliche Solventdampfpartialdruck wäre demzufolge : $P_{OS} = P_{1\,S(T)} / KF1.1$

Falls $KF1.1 < (P_{1.1tot(T)}) / (P_{o.\,tot\,(T)})$ liegt ein Leck in der Messkammer (1) vor, da diese Konstellation physikalisch bei isothermer Kompression nicht möglich ist.

Falls $KF1.1 > (P_{1.1tot(T)}) / (P_{o.\,tot(T)})$ so kondensiert Solventdampf.

Alternative 1

Kompression des Gas/Dampfgemisches bis Kompressionsfaktor (KF1.1) ,bei Gastemperatur $T_1 = T_{1.1} = T_o$

Berechnung des Luftpartialdruckes ($P_{OL}$), des Wasserdampfpartialdruckes ($P_{ow}$) des Solventdampfpartialdruckes (Pos) und des Totaldruckes ($P_{Otot\,(T)}$) in der Messkammer (1) respektiv in der Vakuum -Trockenkammer (12) oder im Probestück (16), gemäss Diagramm (Fig7) diesem Fall durch die Mess/Rechner-Einheit (11) wie folgt: Berechnung von Wasserdampfpartialdruck (Po.w) in Messkammer vor Kompression

$$P_{OW} = (T.P) / KF_{o \div 1.1} = P_{1.1W(T.P)} / KF1.1$$

| Berechnungsbeispiel | :- Kompressionsfaktor KF 1.1 = 60 (A) |
|---|---|
| Vorgabe (A) | - Kompressionsfaktor KF 1. = 5.1 (G) |
| Gemessen (G) | - Taupunkt (T.P): 17.3 °C (G) |
| | - Wasserdampfdruck entsprechend (T.P); $P_{1.1\,w(TP)}$ = 19.7 mbar |
| | -Totaldruck in Messkammer $P_{1.1\,tot(T1.1)}$ = 23 mbar (G) |
| | - Solventdampfdruck bei 20°C ; $P_{1\,s(T1)}$ = 0.655 mbar |

$$P_{OW} = (\,19.7 / 60 = 0.328\text{ mbar}$$

Berechnung des Luftpartialdruckes ($P_{OL}$) in Messkammer vor Kompression

$$P_{OL} = (\,P_{1.1\,tot(T1.1)} - P_{1.1W(T.P)} - P_{1S(T1.2)}\,) / KF1.1$$

$$P_{OL} = (\,23 - 19.7 - 0.655\,) / 60 = 0.044\text{ mbar}$$

Berechnung Totaldruck ($P_{Otot(T)}$) in Messkammer vor Kompression

$$P_{Otot(T)} = ( P_{1.1tot\,(T1.1)} + P_{1.1\,S(T1.1)} \cdot (KF1.1/\,KF1) - P_{1.1\,S(T1.1)} ) / KF1.1$$

$$P_{Otot(T)} = ( 23 + 0.655 \cdot (60/\,5.1) - 0.655 ) / 60 = 0.5\ mbar$$

Berechnung Solventdampfpartialdruck ($P_{OS}$) in Messkammer vor

$$P_{OS}) = 0.5 - 0.044 - 0.328 = 0.128\ mbar$$

Kompression

$$P_{OS}) = P_{Otot(T)} - P_{OL} - P_{OW}$$

$$P_{OS}) = 0.5 - 0.044 - 0.328 = 0.128\ mbar$$

Alternative 1.1

Kompression des Gas/Dampfgemisches bis Kompressionsfaktor (KF1.1) ,bei Gastemperatur $T_1 = T_{1.1} > T_o$
Berechnung des Luftpartialdruckes ($P_{OL}$), des Wasserdampfpartialdruckes (Pow) des Solventdampfpartialdruckes ($P_{OS}$) und des Totaldruckes ($P_{Otot(T)}$) in der Messkammer (1) respektiv in der Vakuum -Trockenkammer (12) oder im Probestück (16), gemäss Diagramm (Fig7) diesem Fall durch die Mess/Rechner-Einheit (11) wie folgt:
Berechnung von Wasserdampfpartialdruck (Po.w) in Messkammer vor Kompression:
In der Formel für Berechnung ($P_{o.w}$) ist infolge der erhöhten Temperatur (T1.1 > To) ebenfalls der gemessene Totaldruck $P_{1.1tot(T1.1)}$ und der Taupunkt (T.P) erhöht.

$$P_{OW} = P_{1.1W(T.P)} \cdot {}^*(To/T_{1.1})^{(\kappa/(\kappa-1))} / KF1.1$$

Berechnungsbeispiel :  - Kompressionsfaktor KF 1.1 = 60 (A)
Vorgabe (A)  - Kompressionsfaktor KF 1. = 9.6 (G)
Gemessen (G)  - Taupunkt (T.P): 19.3 °C (G)
  - Wasserdampfdruck entspechend (T.P); $P_{1.1\,w(TP)}$ = 22.4 mbar
  -Totaldruck in Messkammer $P_{1.1\,tot(1.1)}$ = 26.7 mbar (G)
  - Solventdampfdruck bei 30°C ; $P_{1\,s(T1)}$ = 1.23 mbar
  -Gas/ Dampftemperatur $T_{1.1}$ = 30 °C+ 273° = 303 °K (a)
  -mittl.Adiabatenexponent $\kappa$ = 1.35 (A)

$$P_{OW} = 22.4 \cdot (293/303)^{(1.35/(1.35-1))} / 60 ) = 0.328\ mbar$$

Berechnung Luftpartialdruckes ($P_{OL}$) in Messkammer vor Kompression

$$P_{OL} = (( P_{1.1\,tot(T1.1)} - P_{1.1W(T.P)} ) \cdot {}^*(To/T_{1.1})^{(\kappa/(\kappa-1))} - P_{1S(T1.2)} ) / KF1.1$$

$$P_{OL} = ((\ 26.7 - 22.4)\ *(293/303)^{(1.35/(1.35-1))} - 1.23\ )\ /\ 60 = 0.044\ \text{mbar}$$

Berechnung Totaldruck ($P_{Otot(T)}$) in Messkammer vor Kompression

$$P_{Otot(T)} = ((\ P_{1.1tot\ (T1.1)} + P_{1.1\ S(T1.1)} \cdot (KF1.1/\ KF1 - 1)\ )*(To/T_{1.1})^{(\kappa/(\kappa-1))}\ )/\ KF1.1$$

$$P_{Otot(T)} = ((\ 26{,}7 + 1{,}23 \cdot (60/9{,}6\ -1)\ *(293/303)^{(1.35/(1.35-1))}\ ))\ /\ 60\ )\ = 0.5\ \text{mbar}$$

Berechnung Solventdampfpartialdruck ($P_{OS}$) in Messkammer vor Kompression

$$P_{OS}) = P_{Otot(T)} - P_{OL} - P_{OW}$$

$$P_{OS}) = 0.5 - 0.044 - 0.328 = 0.128\ \text{mbar}$$

Legende L1

| | |
|---|---|
| $P_{O\ tot\ (T)} =$ | Totaldruck in Messkammer (1) nach Druckausgleich mit Vakuum - Trockenkammer (12) und Gas/ Dampftemperatur ($T_O$) |
| $P_{OL} =$ | theoretisch errechneter Luftpartialdruck in Messkammer (1) nach Druckausgleich |
| $P_{OW} =$ | theoretisch errechneter Wasserdampfpartialdruck in Messkammer (1) nach Druckausgleich |
| $P_{OS} =$ | theoretisch errechneter Solventdampfpartialdruck in Messkammer (1) nach Druckausgleich |
| $P_{1tot\ (T)} =$ | gemessener Totaldruck in Messkammer (1) nach Kompression (KF1) und Temperatur $T_1$ |
| $P_{1.1\ tot(T)} =$ | gemessener Totaldruck in Messkammer (1) nach Kompression (KF1.1) und Temperatur T1.1 |
| $P_{1.2\ tot(T)} =$ | gemessener Totaldruck in Messkammer (1) nach Kompression (KF1.2) und Temperatur $T_{1.2}$ |
| $P_{2\ tot(T)} =$ | gemessener Totaldruck in Messkammer (1) nach Kompression (KF2) und Temperatur $T_2$ |
| $P_{1L(T)} =$ | Luftpartialdruck in Messkammer (1) nach Kompression (KF1) und Temperatur $T_O$ |
| $P_{1.1L(T)} =$ | Luftpartialdruck in Messkammer (1) nach Kompression (KF1.1) und Temperatur $T_{1.1}$ |
| $P_{1.2L(T)} =$ | Luftpartialdruck in Messkammer (1) nach Kompression (KF1.2) und Temperatur $T_{1.2}$ |
| $P_{2\ L(T)} =$ | Luftpartialdruck in Messkammer (1) nach Kompression (KF2) und Temperatur $T_2$ |
| $P_{kw(T)} =$ | Kondensationsdruck von Wasserdampf bei Temperatur (T) |
| $P_{1W(T)} =$ | Wasserdampfkondensationdruck in Messkammer (1) nach Kompression (KF1) bei Temperatur ($T_O$ gemäss Wasserdampfdruckkurve |
| $P_{1W} =$ | Wasserdampfpartialdruck in Messkammer (1) nach Kompression (KF1) bei Temperatur $T_1$ |
| $P_{1W\ (TP)} =$ | Wasserdampfpartialdruck in Messkammer (1) nach Kompression (KF1) bei Temperatur $T_1$ gemäss Taupunkt (TP) |
| $P_{1.1W} =$ | Wasserdampfpartialdruck in Messkammer (1) nach 1 Kompression (KF1.1) bei Temperatur $T_{1.1}$ |
| $P_{1.2\ W(T)} =$ | Wasserdampfkondensationdruck in Messkammer (1) nach Kompression (KF1.2) bei Temperatur $T_O$ gemäss Wasserdampfdruckkurve |
| $P_{1.1W(TP)} =$ | Wasserdampfpartialdruck in Messkammer (1) nach Kompression (KF1.1) bei Temperatur $T_{1.1}$ gemäss Taupunkt (TP) |
| $P_{W(TP)M} =$ | Wasserdampfpartialdruck in Messkammer (1) gemäss Taupunktmessung (TP) |
| $P_{1S(T)} =$ | Solventdampfkondesationsdruck in Messkammer (1) nach Kompression (KF1) bei Temperatur T , gemäss Solventdampfdruckkurve |
| $P_{1S(T)} =$ | Kondensationsdruck von Solventdampf bei Temperatur (T) |
| $P_{MAX} =$ | Max. zulässiger Messdruck von Totaldruck Vakuummessgerät Messkammer |
| $T_O =$ | Gas/Dampftemperatur in Messkammer (1) vor Start Kompressionsphase |
| $T_1; T_{1.1}; T_{1.2}; T_2 =$ | Gas/Dampftemperatur entsprechend den Kompressionsstufen (K.F1;KF1.1:KF1.2 KF2) |
| $T_{TPM} =$ | Gas/Dampftemperatur bei Taupunktmessung |
| $T_{GK} =$ | Gas/Dampftemperatur in Messkammer (1) |

| | | |
|---|---|---|
| Ta = | | Gas/Dampftemperatur in Vakuum Saugleitung |
| $T_{OW(P)}$ = | | Kondesationstemperatur Wasserdampf bei Druck (p) |
| $T_{OS(P)}$ = | | Kondesationstemperatur Solventdampf bei Druck (p) |
| KF1 = | | Kompressionsfaktor für Kompressionsstufe = $L_O/L_1$ |
| $L_O$ = | | Position Kompressionskolben (1.1) vor Kompression |
| $L_1$ = | | Kompressionslänge 1. Kompressionsstufe |
| KF1.1 = | | Kompressionsfaktor für Kompressionsstufe $L_O/L_{1.1}$ |
| $L_{1.1}$ = | | Kompressionslänge 1.1 Kompressionsstufe |
| KF1.2 = | | Kompressionsfaktor für Kompressionsstufe = $L_O/L_{1.2}$ bezogen auf Wasserdampf. (Start Kondensation Wasserdampf) |
| $L_{1.2}$ = | | Kompressionslänge 1.2 Kompressionsstufe |
| KF2 = | | Kompressionsfaktor für Kompressionsstufe = $L_O/L_2$ |
| $L_2$ = | | Kompressionslänge 2. Kompressionsstufe |
| $KF_m$ = | | Maximal zulässiger Kompressionsfaktor der Messapparatur |
| $KF2_{WL}$ = | | Kompressionsfaktor für 2. Kompressionsstufe bezogen auf Wasserdampf |
| $KF_{TPM}$ = | | Kompressionsfaktor bei Taupunktmessung |
| $G_{W(h)}$ = | | Stündlicher Wasserentzug aus Vakuum -Trockenkammer (1); (gr/h) |
| S = | | Saugleistung Vakuumpumpen (13;14); ($m_3$/h) |
| $F_K$ = | | Korrelationsfaktor Wasserdampfmenge vs. Wassermenge (gr /$m_3$,mbar) |

Bezugszeichenliste

| | |
|---|---|
| 1 | Messkammer |
| 1A | Vorkompressiomskammer |
| 1.1 | Kompressionskolben mit druck und vakuumfester Dichtung |
| 1.1A | Kompressionskolben mit druck & vakuumfester Dichtung |
| 1.2 | Abschlussdeckel mit druck und vakuumfester Dichtung für Kolbenstange |
| 1.2A | Abschlussdeckel mit Führung für Kolbenstange |
| 1.2.1 | Abschlussdeckel mit mit Führung für Kolbenstange |
| 1.2.2 | Belüftungsstutzen |
| 1.3 | Kolbenstange |
| 1.3A | Kolbenstange |
| 1.4 | Eintrittsöffnung |
| 1.5 | Ablassöffnung |
| 1.5A | Ablass/Zutrittsöffnung |
| 1.6 | Druck-Ausgleichskammer |
| 1.7 | Ein/Austritssöffnung Ausgleichskammer |
| 1.8 | Temperatur Sensor Messkammer |
| 1.9 | Temperatur Sensor Gasraum |
| 2 | Antriebseinheit |
| 2A | Antriebseinheit für Messkammer Vorkompression |
| 2.1 | beweglicher elektronischer Sensor |
| 3 | elektronische Distanzmessung |
| 4 | Kühlelement (Peltier) |
| 5 | Totaldruck Vakuummessgerät für Messkammer |
| 5.1 | Totaldruck Vakuummessgerät für Ausgleichskammer |
| 5.2 | Taupunktmessgerät |
| 6,7,8,9, 10,10A, 10B,10C | Absperrventile |
| 11 | Mess/ Rechner-Einheit |
| 12 | Vakuum-Trockenkammer |
| 12.1 | Trocknungsobjekt |
| 13 | Roots/Kolbenwälzpumpe |
| 14 | Vorvakuumpumpe |
| 15 | Temperatur Sensor Vakuum Saugleitung |
| 16 | Probestück mit innerer Messbohrung |
| 16.1 | Temperatur Sensor Probestück |

17          Verbindungsleitung Probestück zu Trockenkammerwand
18          Verbindungsleitung Trockenkammerwand zu Messapparatur

**Patentansprüche**

1.  Verfahren zur Messung des Partialdrucks ($p_{0L}$, $p_{0W}$) einer Komponente (L, W) eines in einem Trocknungsprozess anfallenden Gemischs aus einem Inertgas (L) und dem Dampf lediglich einer Flüssigkeit (W), bei dem das Gemisch in einer evakuierbaren Messkammer (1) durch Verkleinern des Volumens der Messkammer (1) komprimiert wird und hierbei der Totaldruck des Gemisches erfasst wird, **dadurch gekennzeichnet, dass** der Verlauf des Totaldrucks und der Temperatur des Gemischs in Abhängigkeit von einem Kompressionsfaktor erfasst wird, der durch das Verhältnis der Grösse des Messkammervolumens vor der Kompression zur Grösse des Messkammervolumens nach der Kompression bestimmt wird, und dass
    der Kompressionsvorgang mindestens solange fortgeführt wird bis Kondensation des Dampfs der mindestens einen Flüssigkeit (W) einsetzt, und dass aus hierbei ermittelten Werten des Totaldrucks ($p_{1tot}(T)$, $P_{1.1tot}(T)$), der Temperatur (T) und des Kompressionsfaktors ($KF_1$, $KF_{1.1}$) zumindest beim und/oder nach dem Einsetzen der Kondensation sowie gegebenenfalls dem Totaldruck ($p_{0tot}(T)$) vor der Kondensation und/oder dem Kondensationsdruck ($p_{1w}(T)$) des Dampfs bei der Kondensationstemperatur (T) der Partialdruck ($P_{0L}$, $p_{0w}$) der Komponente (L, W) ermittelt wird.

2.  Verfahren zur Messung des Partialdrucks ($p_{0L}$, $p_{0S}$, $p_{0W}$) einer Komponente (L, S, W) eines in einem Trocknungsprozess anfallenden Gemischs aus einem Inertgas (L), einem Dampf einer höher siedenden Flüssigkeit (S) und einem Dampf einer tiefer siedenden Flüssigkeit (W), **dadurch gekennzeichnet, dass** das Gemisch in einer evakuierbaren Messkammer (1) durch Verkleinern des Volumens der Messkammer (1) mindestens solange komprimiert wird bis Kondensation der höher siedenden Flüssigkeit (S) eintritt und hierbei der Verlauf des Totaldrucks und der Temperatur des Gemischs in Abhängigkeit von einem Kompressionsfaktor erfasst wird, der durch das Verhältnis der Grösse des Messkammervolumens vor der Kompression zur Grösse des Messkammervolumens nach der Kompression bestimmt wird, und dass entweder

    (a) der Kompressionsvorgang mindestens solange fortgeführt wird bis Kondensation des Dampfs der tiefer siedenden Flüssigkeit (W) einsetzt, und dass aus hierbei ermittelten Werten des Totaldrucks ($p_{1tot}(T)$, $p_{1.1tot}(T)$, $P_{1.2tot}(T)$, $p_{2tot}(T)$) und des Kompressionsfaktors ($KF_1$, $KF_{1.1}$, $KF_{1.2}$, $KF_2$) zumindest beim und/oder nach dem Einsetzen der Kondensation des Dampfs der tiefer siedenden Flüssigkeit (W) sowie gegebenenfalls dem Totaldruck ($p_{0tot}(T)$) vor der Kondensation und/oder dem Kondensationsdruck ($p_{1s}(T)$, $P_{1w}(T)$) des Dampfs der höher (S) und/oder des Dampfs der tiefer siedenden Flüssigkeit (W) bei der Kondensationstemperatur (T) der Partialdruck ($P_{0L}(T)$, $p_{0s}(T)$, $p_{0w}(T)$) der Komponente (L, S, W) ermittelt wird,
    oder dass
    (b) am komprimierten Gemisch der Taupunkt des Dampfs der niedriger siedenden Flüssigkeit (W) gemessen und hieraus und aus dem Kompressionsfaktor bei der Taupunktsmessung der Partialdruck ($p_{0W}(T)$) des Dampfs der tiefer siedenden Flüssigkeit (W) ermittelt wird.

3.  Verfahren nach einem der Ansprüche 1 oder 2, **dadurch gekennzeichnet, dass** das Einsetzen der Kondensation durch Überwachung des Verlaufs des Totaldrucks in Abhängigkeit vom Kompressionsfaktor bestimmt wird.

4.  Verfahren nach einem der Ansprüche 1 oder 2, **dadurch gekennzeichnet, dass** der bei der Kompression in der Messkammer (1) aufgebaute Druck durch einen in einer Ausgleichskammer (1.6) wirkenden Gegendruck weitgehend kompensiert wird.

5.  Verfahren nach Anspruch 4, **dadurch gekennzeichnet, dass** das Gemisch vor dem Einbringen in die Messkammer (1) kondensationsfrei komprimiert wird.

6.  Verfahren nach einem der Ansprüche 1 bis 5, **dadurch gekennzeichnet, dass** das Gemisch vor, während und/oder nach der Kompression gekühlt wird.

7.  Verfahren nach einem der Ansprüche 1 bis 6, bei dem das Gas-DampfGemisch beim Trocknen von elektrischen Isolationen und/oder von Apparaten mit elektrischen Isolationen (12.1) in einer Vakuumtrockenkammer (12) anfällt und das Gemisch mit mindestens einer Vakuumpumpe (13, 14) aus der Kammer (12) entfernt wird, **dadurch gekennzeichnet, dass** aus den ermittelten Werten des Partialdrucks des Dampfs ($p_{0W}$) resp. der Partialdrücke ($p_{0S}$, $p_{0W}$) des Dampfs der tiefer (W) und des Dampfs der höher siedenden Flüssigkeit (S) sowie der Saugleistung der Vakuumpumpe die pro Zeiteinheit anfallende Menge an abgepumpter Flüssigkeit resp. an höher und tiefer siedender Flüssigkeit (S, W) ermittelt wird.

8. Verfahren nach einem der Ansprüche 1 bis 7, bei dem das Gas-DampfGemisch beim Trocknen von elektrischen Isolationen und/oder von Apparaten mit elektrischen Isolationen (12.1) in einer Vakuumtrockenkammer (12) anfällt, **dadurch gekennzeichnet, dass** in der Vakuumtrockenkammer (12) zusätzlich mindestens ein Probestück (16) verwendet wird, welches aus dem Material der Isolationen gebildet ist und mindestens einen Hohlraum aufweist, der zur Messung des Partialdrucks ($p_{0L}$, $p_{0S}$, $p_{0W}$) mindestens einer der Komponenten (L, S, W) eines im Hohlraum befindlichen Gas-Dampf-Gemischs mit der Messkammer (1) verbunden wird.

9. Vorrichtung zur Durchführung des Verfahrens nach einem der Ansprüche 1 bis 8, enthaltend :

   - eine evakuierbare Messkammer (1), deren Volumen durch die Position eines lageveränderlichen Kolbens (1.1) bestimmt ist,
   - mindestens eine Anschlussöffnung (1.4, 1.5), über den die Messkammer (1) mit einer Vakuum-Trockenkammer (12) verbindbar ist, welche zur Aufnahme des im Trocknungsprozess anfallende Gemisch vorgesehen ist,
   - ein erstes Absperrventil (10), welches zwischen der Messkammer (1) und der Vakuum -Trockenkammer (12) angeordnet ist,
   - ein den Totaldruck in der Messkammer (1) erfassendes Vakuummessgerät (5), und
   - einen die Temperatur in der Messkammer (1) erfassenden Sensor (1.8,1.9), **gekennzeichnet**
   - **durch** einen die Position des Kolbens (1.1) erfassenden elektronischen Distanzmesssensor (2.1,3) zur Ermittlung eines Kompressionsfaktors, der durch das Verhältnis der Grösse des Messkammervolumens vor einer Kompression des Gemisches zur Grösse des Messkammervolumens nach der Kompression bestimmt ist, und
   - **durch** eine Ausgangssignale des Totaldruckssensors (5), des Temperatursensors (1.8,1.9) und des elektronischen Distanzmessers (2.1,3) erfassende und verarbeitende Rechnereinheit (11) zur Ermittlung des Partialdrucks.

10. Vorrichtung nach Ansprüche 9, **dadurch gekennzeichnet, dass** der Kolben (1.1) zwei Arbeitsräume begrenzt, von denen der eine, die Messkammer (1) und der andere eine Druckausgleichskammer (1.6) ist.

11. Vorrichtung nach einem der Ansprüche 9 oder 10, **dadurch gekennzeichnet, dass** die Messkammer (1) ein temperaturüberwachtes Kühlelement (4) enthält.

12. Vorrichtung nach einem der Ansprüche 9 bis 11, **dadurch gekennzeichnet, dass** die Messkammer (1) über die Anschlussöffnung (1.5) und ein zweites Absperrventil (10A) mit einer Vorkompressionskammer (1A) verbindbar ist.

13. Vorrichtung nach Anspruch 12, **dadurch gekennzeichnet, dass** parallel zur ersten Vorkompressionskammer (1A) mindestens eine zweite Vorkompressionskammer (1A) geschaltet ist.

**Claims**

1. A method for measuring the partial pressure, $p_{0L}$, $p_{0W}$) of a component (L, W) of a mixture of an inert gas (L) and the vapour of only one liquid (W) produced in a drying process, wherein the mixture is compressed in an evacuable measuring chamber (1) by reducing the volume of the measuring chamber (1), and where the total pressure of the mixture is recorded, **characterised in that** the curve of the total pressure and the temperature of the mixture is recorded as a function of a compression factor which is determined by the ratio of the size of the measuring chamber volume before the compression to the size of the measuring chamber volume after the compression, **in that** the compression process is continued until condensation of the vapour of the at least one liquid (W) stops, and **in that** the partial pressure ($p_{0L}$, $p_{0W}$) of the component (L, W) is determined from values of the total pressure ($p_{1tot}$ (T), $P_{1.1tot}$ (T)) determined hereby, the temperature (T) and the compression factor ($KF_1$, $KF_{1.1}$) at least during and/or after stopping of the condensation and, if necessary, from the total pressure ($p_{0tot}$ (T)) before the condensation and/or the condensation pressure ($p_{1w}$ (T)) of the vapour at the condensation temperature (T).

2. The method for measuring the partial pressure ($p_{0L}$, $P_{0S}$, $P_{0W}$) of a component (L, S, W) of a mixture of an inert gas (L), a vapour of a higher boiling liquid (S) and a vapour of a lower boiling liquid (W) produced in a drying process, **characterised in that** the mixture is compressed in an evacuable measuring chamber (1) by reducing the volume of the measuring chamber(1) at least until condensation of the higher boiling liquid (S) commences, and where the curve of the total pressure and of the temperature of the mixture is recorded as a function of a compression factor which is determined by the ratio of the size of the measuring chamber volume before the compression to the size of the measuring chamber volume after the compression, and **in that** either

(a) the compression process is continued at least until condensation of the vapour of the lower boiling liquid (W) commences, and **in that**
the partial pressure ($p_{0L}$ (T), $p_{0S}$ (T), $p_{0W}$ (T)) of the component (L, S, W) is determined from values of the total pressure ($p_{1tot}$ (T), $p_{1.1tot}$(T), $p_{1.2}$(T), $p_{2tot}$(T)) and of the compression factor ($KF_1$, $KF_{1.1}$, $KF_{1.2}$, $KF_2$) obtained thereby at least during and/or after commencement of the condensation of the vapour of the lower boiling liquid (W) and, if necessary, the total pressure ($p_{2tot}$(T)) before the condensation and/or the condensation pressure ($p_{1S}$(T), $p_{1w}$(T)) of the vapour of the higher (S) and/or the vapour of the lower boiling liquid W) at the condensation temperature (T),
or that
(b) the dew point of the vapour of the lower boiling liquid (W) is measured on the compressed mixture, and the partial pressure ($p_{0W}$ (T)) of the vapour of the lower boiling liquid (W) is determined from this and from the compression factor during the dew point measurement.

3. The method according to one of Claims 1 or 2, **characterised in that** the commencement of the condensation is determined by monitoring the curve of the total pressure as a function of the compression factor.

4. The method according to one of Claims 1 or 2, **characterised in that** the pressure developed during compression in the measuring chamber (1) is largely compensated for by a counterpressure acting in an equalisation chamber (1.6).

5. The method according to Claim 4, **characterised in that** the mixture is compressed free from condensation before being introduced into the measuring chamber (1).

6. The method according to Claims 1 to 5, **characterised in that** the mixture is cooled before, during and/or after compression.

7. The method according to one of Claims 1 to 6, wherein the gas-vapour mixture is produced in a vacuum drying chamber (12) when drying electrical insulations and/or apparatus with electrical insulations (12.1), and the mixture is removed from the chamber with at least one vacuum pump (13, 14), **characterised in that** the quantity of pumped off liquid produced per unit of time, or the quantity of higher and lower boiling liquid (S, W), is determined from the determined values of the partial pressure of the vapour ($p_{0W}$) and the partial pressures ($p_{0s}$, $p_{0w}$) of the vapour of the lower (W) and of the vapour of the higher boiling liquid (S) respectively, and from the suction power of the vacuum pump.

8. The method according to one of Claims 1 to 7, wherein the gas-vapour mixture is produced during drying of electrical insulations and/or of apparatus with electrical insulations (12.1) in a vacuum drying chamber (12), **characterised** that at least one test piece (16) is also used in the vacuum drying chamber (12), which test piece is formed from the material of the insulations and has at least one cavity which is connected to the measuring chamber (1) to measure the partial pressure ($p_{0L}$, $p_{0S}$, $p_{0W}$) of at least one of the components (L, S, W) of a gas-vapour mixture present in the cavity.

9. A device for carrying out the method according to one of Claims 1 to 8, comprising:

- an evacuable measuring chamber (1) whose volume is determined by the position of a position-variable piston (1.1),
- at least one connection opening (1.4, 1.5) by means of which the measuring chamber(1) can be connected to a vacuum drying chamber (12) which is provided for receiving the mixture produced in the drying process,
- a first shutoff valve (10) which is arranged between the measuring chamber (1) and the vacuum drying chamber (12),
- a vacuum measuring device (5) recording the total pressure in the measuring chamber(1), and
- a sensor (1.8, 1.9) recording the temperature in the measuring chamber (1), **characterised**
- **by** an electronic distance sensor (2.1,3) that records the position of the piston (1.1) for determining a compression factor which is determined by the ratio of the size of the measuring chamber volume before a compression of the mixture to the size of the measuring chamber volume after the compression, and
- by a computer unit (11) recording and processing an output signal of the total pressure sensor (5), the temperature sensor (1.8, 1.9) and the electronic distance meter (2.1,3) for determining the partial pressure.

10. The device according to Claim 9, **characterised in that** the piston (1.1) delimits two working spaces, one of which is the measuring chamber (1) and the other is a pressure equalisation chamber (1.6).

11. The device according to one of Claims 9 or 10, **characterised in that** the measuring chamber (1) contains a temperature-monitored cooling element (4).

12. The device according to one of Claims 9 to 11, **characterised in that** the measuring chamber (1) can be connected to a pre-compression chamber (1A) by means of the connection opening (1.5) and a second shutoff valve (10A).

13. The device according to Claim 12, **characterised** that at least one second pre-compression chamber is connected in parallel to the first pre-compression chamber (1A).

**Revendications**

1. Procédé pour mesurer la pression partielle ($P_{OL}$, $P_{OW}$) d'un composant (L, W) d'un mélange produit dans un processus de séchage constitué d'un gaz inerte (L) et de la vapeur uniquement d'un liquide (W), dans lequel le mélange est comprimé dans une chambre de mesure (1) dans laquelle on peut faire le vide par la réduction du volume de la chambre de mesure (1) et la pression totale du mélange est alors enregistrée, **caractérisé en ce que** la courbe de la pression totale et de la température du mélange est enregistrée en fonction d'un facteur de compression qui est déterminé par le rapport entre la grandeur du volume de la chambre de mesure avant la compression et la grandeur du volume de la chambre de mesure après la compression, et **en ce que**
l'opération de compression est poursuivie au moins jusqu'à ce que la condensation de la vapeur du au moins un liquide (W), commence, et
**en ce que** la pression partielle ($P_{OL}$, $P_{Ow}$) du composant du (L, W) est déterminée à partir de valeurs déterminées à cette occasion de la pression totale ($P_{1tot}$ (T), $P_{1.1tot}$ (T)) de la température (T) du facteur de compression ($KF_1$, $KF_{1.1}$) au moins lors du commencement et/ou après le commencement de la condensation et éventuellement après la pression totale ($P_{0T}$ (T)) avant la condensation et/ou la pression de condensation ($P_{tw}$ (T)) de la vapeur à la température de condensation (T).

2. Procédé pour mesurer la pression partielle ($P_{OL}$, $P_{OS}$, $P_{Ow}$) d'un composant (L, S, W) d'un mélange produit dans un processus de séchage constitué d'un gaz inerte (L), d'une vapeur d'un liquide (S) à point d'ébullition assez élevé et d'une vapeur d'un liquide (W) à point d'ébullition assez bas, **caractérisé en ce que** le mélange est comprimé dans une chambre de mesure (1) dans laquelle on peut faire le vide par la réduction du volume de la chambre de mesure (1) au moins jusqu'à ce que la condensation du liquide (S) à point d'ébullition assez élevé survienne et que la courbe de la pression totale et de la température du mélange soit enregistrée à cette occasion en fonction d'un facteur de compression, qui est déterminé par le rapport entre la grandeur du volume de la chambre de mesure avant la compression et la grandeur du volume de la chambre de mesure après la compression, et **en ce que** soit

a) l'opération de compression est poursuivie au moins jusqu'à ce que la condensation de la vapeur du liquide (W) à point d'ébullition assez bas commence, et **en ce que**
la pression partielle ($P_{OL}$ (T), $P_{OS}$ (T), $P_{OW}$ (T)) du composant (L, S, W) est déterminée à partir de valeurs calculées dans le cas présent de la pression totale ($P_{1tot}$ (T), $P_{1.1tot}$ (T), $P_{1.2tot}$ (T), $P_{2tot}$ (T)) et du facteur de compression ($KF_1$, $KF_{1.1}$, $KF_{1.2}$, $KF_2$) au moins lors du début et/ou après le début de la condensation de la vapeur du liquide (W) à point d'ébullition assez bas et éventuellement après la pression totale ($P_{0tot}$ (T)), avant la condensation et/ou la pression de compression ($P_{1S}$ (T), $P_{1W}$ (T)) de la vapeur du liquide (S) à point d'ébullition assez élevé et/ou de la vapeur du liquide (W) à point d'ébullition assez bas à la température de condensation (T), ou **en ce que**
(b) le point de rosée de la vapeur du liquide (W) à point d'ébullition assez bas est mesuré sur le mélange comprimé et la pression partielle ($P_{OW}$ (T)) de la vapeur du liquide (W) au point d'ébullition assez bas est déterminée à partir de là et à partir du facteur de compression lors de la mesure du point de rosée.

3. Procédé selon l'une quelconque des revendications 1 ou 2, **caractérisé en ce que** le début de la condensation est déterminé par le contrôle de la courbe de la pression totale en fonction du facteur de compression.

4. Procédé selon l'une quelconque de la revendication 1 ou 2, **caractérisé en ce que** la pression établie lors de la compression dans la chambre de mesure (1) est compensée largement par une contre-pression agissant dans une chambre d'équilibrage (1.6).

5. Procédé selon la revendication 4, **caractérisé en ce que** le mélange est comprimé sans condensation avant l'introduction dans la chambre de mesure (1).

6. Procédé selon l'une quelconque des revendications 1 à 5, **caractérisé en ce que** le mélange est refroidi avant, pendant ou après la compression.

7. Procédé selon l'une quelconque des revendications 1 à 6, dans lequel le mélange gaz-vapeur est occasionné lors du séchage d'isolations électriques et/ou d'appareils avec des isolations (12.1) électriques dans une chambre de séchage sous vide (12) et le mélange est enlevé avec au moins une pompe à vide (13, 14) de la chambre (12), **caractérisé en ce que**, à partir des valeurs déterminées des pression partielle de la vapeur ($P_{OW}$) et des pressions partielles ($P_{OS}$, $P_{OW}$) de la vapeur du liquide à point d'ébullition assez bas (W) et de la vapeur du liquide (S) à point d'ébullition assez élevé ainsi que de la puissance d'aspiration de la pompe à vide, on détermine la quantité produite par unité de temps de liquide évacué par pompage respectivement de liquide (S, W) à point d'ébullition assez élevé et assez bas.

8. Procédé selon l'une quelconque des revendications 1 à 7, dans lequel le mélange gaz-vapeur est produit lors du séchage d'isolations électriques ou d'appareils avec des isolations (12.1) électriques dans une chambre de séchage sous vide (12), **caractérisé en ce que**, dans la chambre de séchage sous vide (12), on utilise en supplément au moins un échantillon (16) qui est formé dans le matériau des isolations et présente au moins une cavité qui est reliée à la chambre de mesure (1) pour la mesure de la pression partielle ($P_{OL}$, $P_{OS}$, $P_{OW}$) d'au moins l'un des composants (L, S, W) d'un mélange gaz-vapeur se trouvant dans la cavité.

9. Dispositif pour la mise en oeuvre du procédé selon l'une quelconque des revendications 1 à 8, contenant :

   - une chambre de mesure (1), dans laquelle on peut faire le vide, dont le volume est déterminé par la position d'un piston (11) variable en position,
   - au moins une ouverture de raccordement (1.4, 1.5), par laquelle la chambre de mesure (1) peut être reliée à une chambre de séchage sous vide (12), qui est prévue pour le logement du mélange produit dans le processus de séchage,
   - une première vanne d'arrêt (10), qui est disposée entre la chambre de mesure (1) et la chambre de séchage sous vide (12),
   - un appareil de mesure de vide (5) enregistrant la pression totale dans la chambre de mesure (1), et
   - un capteur (1.8, 1.9) enregistrant la température dans la chambre de mesure (1),

   **caractérisé**

   - **par** un capteur de mesure de distance (2.1,3) électronique, enregistrant la position du piston (1.1) pour la détermination d'un facteur de compression qui est déterminé par le rapport entre la grandeur du volume de la chambre de mesure avant une compression du mélange et la grandeur du volume de la chambre de mesure après la compression, et
   - par une unité de calcul (11) enregistrant et traitant des signaux du capteur de pression totale (5), du capteur de température (1.8, 1.9) et du mesureur de distance (2.1.3) électronique pour la détermination de la pression partielle.

10. Dispositif selon la revendication 9, **caractérisé en ce que** le piston (1.1) présente deux espaces de travail, dont l'un est la chambre de mesure (1) et l'autre une chambre d'équilibrage de pression (1.6).

11. Dispositif selon l'une quelconque des revendications 9 ou 10, **caractérisé en ce que** la chambre de mesure (1) contient un élément de refroidissement (4) contrôlé par température.

12. Dispositif selon l'une quelconque des revendications 9 à 11, **caractérisé en ce que** la chambre de mesure (1) peut être reliée par l'ouverture de raccordement (1.5) et par une seconde vanne d'arrêt (10A) à une chambre de précompression (1A).

13. Dispositif selon la revendication 12, **caractérisé en ce qu'**au moins une seconde chambre de précompression (1A) est branchée parallèlement à la première chambre de précompression (1A).

# Fig 1

HVM1

# Fig.2

HVM1

# Fig 3

HVM1

# Fig 4

HVM1

# Fig 5

HVM1

## Fig.6

## Fig.7

HVM1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CH 469259 A
- US 2715836 A